# EUROPÄISCHE PATENTSCHRIFT

(11) **EP 2 771 316 B1**
(45) Veröffentlichungstag und Bekanntmachung des Hinweises auf die Patenterteilung: **16.10.2019**
(21) Anmeldenummer: 12780184.3
(22) Anmeldetag: 29.10.2012
(51) Int. Cl.: C07C 275/14, C07C 279/16, A61K 31/155, A61P 31/16

(54) **VERBINDUNGEN ZUR THERAPIE DER INFLUENZA**
COMPOUNDS FOR THE TREATMENT OF INFLUENZA
COMPOSÉS PERMETTANT DE TRAITER LA GRIPPE

(30) Priorität: 28.10.2011 DE 102011117128
(43) Veröffentlichungstag der Anmeldung: 03.09.2014
(73) Patentinhaber: Christian-Albrechts-Universität zu Kiel, 24118 Kiel (DE)
(72) Erfinder: CLEMENT, Bernd, 24106 Kiel (DE); KOTTHAUS, Joscha, 24116 Kiel (DE); KOTTHAUS, Jürke, 24118 Kiel (DE); SCHADE, Dennis, La Jolla, CA 92037 (US)
(74) Vertreter: Steinecke, Peter
(86) Internationale Anmeldenummer: PCT/EP2012/071382
(87) Internationale Veröffentlichungsnummer: WO 2013/060889

(56) Entgegenhaltungen:
- EP-A1- 2 415 471
- WO-A1-2009/146320
- WO-A1-2010/075636
- WO-A1-2011/123856
- WO-A2-2009/029888
- DE-A1-102009 008 256
- KUNG-CHENG LIU ET AL: "Intramolecular ion-pair prodrugs of zanamivir and guanidino-oseltamivir", BIOORGANIC & MEDICINAL CHEMISTRY, PERGAMON, GB, Bd. 19, Nr. 16, 27. Juni 2011 (2011-06-27) , Seiten 4796-4802, XP028252898, ISSN: 0968-0896, DOI: 10.1016/J.BMC.2011.06.080 [gefunden am 2011-07-01]
- WILLIAMS M A ET AL: "Structure-activity relationships of carbocyclic influenza neuraminidase inhibitors", BIOORGANIC & MEDICINAL CHEMISTRY LETTERS, PERGAMON, ELSEVIER SCIENCE, GB, Bd. 7, Nr. 14, 22. Juli 1997 (1997-07-22), Seiten 1837-1842, XP004136342, ISSN: 0960-894X, DOI: 10.1016/S0960-894X(97)00333-8
- KIM C U ET AL: "Structure-activity relationship studies of novel carbocyclic influenza neuraminidase inhibitors", JOURNAL OF MEDICINAL CHEMISTRY, AMERICAN CHEMICAL SOCIETY, US, Bd. 41, Nr. 14, 9. Juni 1998 (1998-06-09), Seiten 2451-2460, XP002389168, ISSN: 0022-2623, DOI: 10.1021/JM980162U
- JIUN-JIE SHIE ET AL: "Synthesis of Tamiflu and its Phosphonate Congeners Possessing Potent Anti-Influenza Activity", JOURNAL OF THE AMERICAN CHEMICAL SOCIETY, Bd. 129, Nr. 39, 1. Oktober 2007 (2007-10-01), Seiten 11892-11893, XP55051170, ISSN: 0002-7863, DOI: 10.1021/ja073992i in der Anmeldung erwähnt

## Beschreibung

Die vorliegende Erfindung betrifft neue Inhibitoren der Neuraminidase zur Therapie der Influenza (Grippe) und Verfahren zu deren Herstellung.

Influenza stellt eine ernste Virusinfektion des Respirationstraktes dar. Allein in den USA wird angenommen, dass sich jährlich zwischen 10% und 20% der Bevölkerung mit diesem Virus infizieren. Nach Angaben der WHO ist dieses Virus verantwortlich für die Erkrankung von 3 bis 5 Millionen Menschen und 250.000 bis 500.000 Todesfälle pro Jahr, verursacht entweder direkt durch das Virus oder durch Sekundär-Infektionen. Durch unregelmäßig auftretende Epidemien oder Pandemien werden die Infektionszahlen sowie die Todesfälle deutlich erhöht. Im letzten Jahrhundert haben drei große Influenza-Pandemien, ein H1N1-Virus in den Jahren 1918-19 ("Spanische Grippe"), ein H2N2-Virus 1957 und ein H3N2-Virus 1968 kumulativ etwa 50 Millionen Todesfälle verursacht. Die schwerste Pandemie stellte dabei bisher die "Spanische Grippe" dar, durch die alleine im 1. Jahr etwa 20 Millionen Menschen getötet wurden. Die letzte Pandemie erfolgte 2009 durch die "Mexiko-Grippe" (H1N1, "Schweine-Grippe"), die jedoch gemessen an Todesfällen vergleichsweise harmlos verlaufen ist. Insbesondere jedoch das hochpathogene "Vogelgrippe-Virus" (H5N1) gibt in den letzten Jahren Anlass zur Besorgnis, u.a. auch wegen der leichten Übertragung von Tier auf Mensch.

Derzeit gibt es zwei prinzipielle (und zugelassene) therapeutische Ansätze, um die Grippe zu behandeln, bzw. einer Infektion vorzubeugen:
(1) Die Impfung
(2) Der Einsatz von antiviralen Arzneistoffen:
   a. M2-Kanal Blocker, Adamantan-Derivate (Amantadin, Rimantadin)
   b. Neuraminidase Inhibitoren

Neuraminidase-Inhibitoren weisen allerdings entscheidende Vorteile gegenüber den M2-Kanal-Blockern auf:
(1) ein breites Spektrum antiviraler Wirksamkeit, da wirksam gegen Influenza A und B. Die Adamantan-Derivate wirken nur gegen Influenza A;
(2) eine weniger starke Induktion viraler Resistenzmechanismen;
(3) bessere Verträglichkeit;
(4) bessere Wirksamkeit respiratorische Events zu reduzieren.

Das Influenza-Virus besteht aus einer äußeren Membran, die das Nucleocapsid umgibt. Auf dieser äußeren Membran befinden sich die Glykoproteine Hemagglutinin und Neuraminidase. Nachdem das Virus die Zellen befallen hat und die Virus Replikation initiiert wurde, werden neue Virus Partikel, sogenannte Virions, gebildet, die mit Sialinsäure ummantelt sind. Solange die Sialinsäure an den Virions gebunden ist, aggregieren sie mit den Hemagglutinin Resten anderer Virions. Diese Virion-Aggregationen sind nicht mehr in der Lage, in andere Zellen einzudringen und sie zu infizieren. Daher ist eine der Funktionen der Neuraminidase die Abspaltung der Sialinsäure-Reste von den Virions, damit diese frei im Körper zirkulieren und andere Zellen infizieren können. Somit stellt der Einsatz von Neuraminidase Inhibitoren einen Therapieansatz in der Behandlung der Influenza dar.

Mittlerweile wurden einige sehr potente Inhibitoren der Neuraminidase wie das Zanamivir, das Oseltamivir und das Peramivir entwickelt, die die pharmazeutische Zulassung zur Therapie der Influenza erhalten haben. Des Weiteren, beschreibt WO 2011/123856 A1 Neuraminidaseinhibitoren.

Auf dem Gebiet der Neuraminidase Inhibitoren wurde viel Forschungs- und Entwicklungs-Arbeit geleistet, um neue potentielle Arzneistoffe zu identifizieren. Neben der Entwicklung neuer Leitstrukturen wurden zahlreiche Ansätze verfolgt, deren Ziel die Optimierung bzw. Modifizierung der bekannten Strukturen von Oseltamivir und Zanamivir war. Gemeinsames Ziel aller Arbeiten war stets, Verbindungen mit entweder verbesserter Wirksamkeit oder Bioverfügbarkeit zu erhalten. Dabei ist es nicht gelungen, eine Verbindung zu entwickeln, die beide Kriterien vollständig erfüllt. Jedoch wurde eine Vielzahl an Modifikationen für das Oseltamivir und das Zanamivir beschrieben, welche zu ebenfalls gut wirksamen Verbindungen gegenüber der Influenza führen. Eine Übersicht über die bereits beschriebenen Modifikationen ist im Folgenden gegeben:

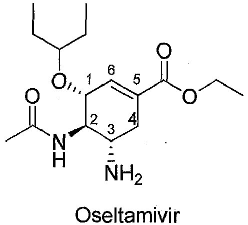

### Position 1:

Viele Arbeiten beziehen sich auf die Abwandlung der Isopentyl-Seitenkette. Inzwischen sind zahlreiche Modifikationen bekannt, die der Erniedrigung oder Erhöhung der Lipophilie durch modifizierte Seitenketten dienen und dadurch die Affinität zur Neuraminidase nur geringfügig beeinflussen.¹ Zudem konnte gezeigt werden, dass eine Hydroxylierung an jeder Position in der Isopentylkette möglich ist.² Auf Basis des strukturell ähnlichen Zanamivirs wurde gezeigt, dass auch verschieden substituierte aliphatische Reste, sowie verschiedene zyklische Seitenketten (hydroxyliert und unhydroxyliert) in dieser Position akzeptiert werden.3 Li *et al.* erweiterten diesen Ansatz mit dem rationalen Design 46 Oseltamivir analoger Verbindungen und zeigten, dass eine Substitution in 1 Position sinnvoll ist, um die Affinität zur Neuraminidase zu erhöhen. Auch in dieser Studie konnten verschiedene Substituenten - sowohl aromatischer als auch aliphatischer Natur - identifiziert werden. Ferner konnte der Sauerstoff durch einen Stickstoff ersetzt werden.4 Diese Daten belegen, dass die strukturellen Anforderungen an dieser Molekülposition vergleichsweise unspezifisch sind und verschiedenste Strukturen akzeptiert werden. Durch Modifikation der Isopentylfunktion sind somit zahlreiche weitere wirksame Derivate des Oseltamivirs darstellbar, die durch die erfindungsgemäßen nachfolgend beschriebenen Verfahren erhalten werden können.

### Position 4:

Die Modifikation der 4-Position des Oseltamivirs wurde beispielsweise von Wang *et al.* untersucht.⁶ Dabei wurde gezeigt, dass eine Substitution in dieser Position möglich ist, um die Affinität zur Neuraminidase zu optimieren. Die Substituenten wurden sowohl über Kohlenstoff, Sauerstoff bzw. Stickstoff mit dem Cyclohexen-Grundgerüst verknüpft.

### Position 5:

In dieser Position sind ebenfalls verschiedene Modifikationen möglich. So konnte beispielsweise gezeigt werden, dass die Carboxylfunktion durch andere analoge Funktionen (Phosphonsäuren, Posphonsäureester, Phosponsäureamide, etc.), ausgetauscht werden kann ohne ihre Wirksamkeit zu verlieren.^{7, 8} Der Austausch des Carboxyl-Kohlenstoffs durch einen Schwefel zu den entsprechenden Sulfonsäure-Derivaten ist ebenso denkbar.

### Position 6 und Grundgerüst:

Weitere Modifikationen beziehen sich auf das Cyclohexen Grundgerüst des Oseltamivirs. Verschiedene Arbeitskreise beschäftigten sich damit, dieses zentrale Strukturmerkmal durch andere Strukturen zu ersetzen, wobei an der zyklischen Struktur des Elements nichts verändert wurde. Es zeigte sich, dass auch aromatisierte Systeme sowie 5-gliedrige Ringsysteme über ähnliche Wirksamkeiten verfügen können.^{9, 10} Zudem wurden verschiedene aliphatische Substitutionen in 6 Position getestet und deren Affinität zur Neuraminidase gezeigt. Ebenso denkbar ist der Einsatz von Hetero-Atomen in das Ringsystem, wie es beim Zanamivir erfolgt ist. Die Modifikationen im Grundgerüst belegen damit, dass nicht nur die Größe des zentralen Strukturelements variiert werden kann, sondern auch, das die Doppelbindung im Cyclohexen des Oseltamivirs nicht essentiell ist. Sowohl Variationen in der Anzahl als auch in der Position der Doppelbindungen führt zu potenten Neuraminidase Inhibitoren.^{10, 11}

Der große Entwicklungseinsatz, der auf dem Gebiet der Entwicklung von Neuraminidase Inhibitoren erfolgt ist, führte zur Identifikation von zahlreichen Oseltamivir analogen Strukturen, die über eine ähnliche Wirksamkeit wie das Oseltamivir verfügen. In der Summe ihrer Eigenschaften ist jedoch keines dieser Derivate dem Oseltamivir überlegen, so dass für keines dieser Analoga eine Zulassung als Arzneistoff erfolgt ist.

Entscheidend für den Erfolg eines Therapeutikums ist sein pharmakokinetisches Profil, welches durch Löslichkeit, Aktivierbarkeit möglicher Prodrugs in ihre Wirkformen sowie das Ausbilden von ProteinBindungen, beeinflusst wird. Ebenso wichtig ist seine Stabilität unter physiologischen Bedingungen.

Von entscheidender Bedeutung ist zudem die orale Bioverfügbarkeit eines Therapeutikums, Medikaments oder Wirkstoffs, welche das Ausmaß angibt, in dem ein Arzneistoff nach oraler Applikation absorbiert wird. Die orale Applikation ist verglichen mit anderen Applikationsformen am einfachsten anzuwenden. So kann beispielsweise auf Sterilität, wie sie bei der Infusion oder Injektion benötigt wird, verzichtet werden. Zudem ist die Dosierung und Anwendung deutlich einfacher als die bei einer inhalativen Arzneiform.

Zurzeit sind in Deutschland das Zanamivir und das Oseltamivir zur Behandlung von Influenza Infektionen zugelassen. Während für Oseltamivir bereits eine orale Darreichungsform zur Verfügung steht (Tamiflu®), ist der Einsatz von Zanamivir nur inhalativ möglich (Relenza®), da die Bioverfügbarkeit nach oraler Applikation lediglich 2% beträgt. Zusätzlich ist in anderen Ländern das Peramivir als Notfallmedikament zur intravenösen Applikation im Falle einer Pandemie zugelassen. Kürzlich wurde Laninamivir (Inavir®) zur inhalativen Therapie der Influenza in Japan zugelassen.

Im Falle einer Pandemie zeigt sich, dass die oralen Darreichungsformen gegenüber den inhalativen oder intravenösen von Vorteil sind.

Ein sehr großes Problem der anti-Influenza-Wirkstoffe ist die schnelle Mutation der Influenza-Viren, insbesondere der viralen Hauptantigene (Hämagglutinin und Neuraminidase), so dass sich rasch Resistenzen gegen die vorhandenen Medikamente ausbilden. Diese Mutation ist bei Influenza A Viren besonders ausgeprägt. Es kann zu einem plötzlichen sogenannten "antigenen Shift" kommen, der die jährliche (saisonale) Anpassung der Impfstoffe gegen Influenza erforderlich macht. Insbesondere die verstärkte Inzidenz der Oseltamivir-Resistenz gegenüber A(H1N1) mit der H274Y Mutation in den letzten Jahren gibt Anlass zur Besorgnis. Des Weiteren wurden neben einigen bekannten Mutationen weitere neue Resistenzmechanismen entdeckt.

Sollte durch weitergehende Resistenzen des Virus Oseltamivir (Tamiflu®) unwirksam werden, geht eine wertvolle oral zu applizierende Substanz mit Wirksamkeit gegen Influenza verloren.

Es ist daher Aufgabe der Erfindung ausgehend von der Oseltamivir-Struktur Neuraminidase Inhibitoren zu finden, die auch gegenüber Oseltamivir-resistenten Virenstämmen wirksam sind.

Weiterhin ist es Aufgabe der Erfindung Neuraminidase Inhibitoren bereitzustellen, welche über ein verbessertes pharmakokinetisches Profil verfügen.

Des Weiteren ist es Aufgabe der Erfindung Neuraminidase Inhibitoren bereitzustellen, welche über eine gut Bioverfügbarkeit verfügen.

Die Aufgabe wird durch die in den Ansprüchen und der vorliegenden Beschreibung gekennzeichneten Ausführungsformen gelöst. Die Unteransprüche und Beispiele geben vorteilhafte Ausgestaltungen der Erfindung an.

Die Aufgabe der Erfindung wird gelöst durch
[1.] Verbindung gemäß der allgemeinen Strukturformel 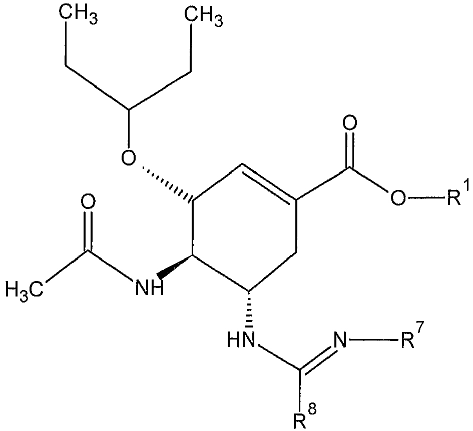 sowie pharmazeutisch verträgliche Salze, Solvate und/oder R/S Enatiomere hiervon, wobei
   R¹ H, eine verzweigte oder unverzweigte, gesättigte oder ungesättigte, substituierte, oder nicht substituierte Kohlenwasserstoffkette mit einer Kettenlänge von 1 bis 12 ist,
   R⁷ H oder OH ist,
   R⁸ H, R⁹, NH₂, NHR⁹, N(R⁹)₂ oder NHCOOR¹ ist,
   R⁹ ein verzweigter oder unverzweigter Alkylrest mit einer Kettenlänge von 1 bis 4 C-Atomen ist, und
   wobei mögliche Substituenten für R¹ und R⁹, ausgewählt sind aus einer Gruppe bestehend aus Fluor, Chlor, Brom und Jod, Sauerstoff, Schwefel, Alkoxy-, Acyloxy-, Hydroxyl-, Mercapto-, Cyano-, Nitro- und Thioalkoxy-Gruppe,
   wobei wenn R⁷ H und R⁸NH₂ ist, R¹ nicht H oder CH₂CH₃ ist.
[2.] Verbindung nach [1], wobei die Verbindung Amidoxim (3*R*,4*R*,5*S*)-4-Acetamido-5-[*N*-(*N*'-hydroxy)acetimidamido]-3-(1-ethylpropoxy)cyclohex-1-en-1-carbonsäureethylester oder (3R,4R,5S)-4-Acetamido-5-(N-acetimidamido)-3-(1-ethylpropoxy)cyclohex-1-en-1-carbonsäure und/oder pharmazeutisch verträgliche Salze, Solvate und/oder R/S Enantiomere hiervon ist.
[3.] Verbindung nach [1] oder [2] zur Verwendung als Arzneimittel.
[4.] Verbindung gemäß [1] oder [2] zur Behandlung von Influenza Infektionen.
[5.] Verfahren zur Herstellung einer Verbindung nach [1] oder [2] umfassend die Umsetzung von Oseltamivir gemäß der allgemeinen Strukturformel 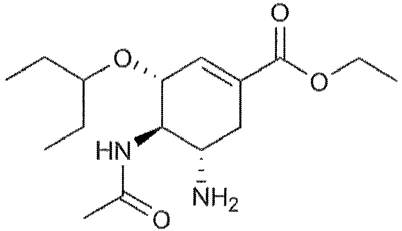 oder eines Derivates davon, welches mindestens den folgenden Schritt umfasst:
   Umsetzung des Oseltamivirs mit einem Acylhydroximoylchlorid, in organischen Lösungsmitteln bei Raumtemperatur.
[6.] Verfahren nach [5], wobei das organische Lösungsmittel ein Dichlormethan ist.
[7.] Verfahren zur Herstellung einer Verbindung nach einem der [1] bis [3] umfassend die Umsetzung von Oseltamivir (3R,4R,5S)-4-Acetamido-5-amino-3-(1-ethylpropoxy)cyclohex-1-en-1-carbonsäureethylester oder eines Derivates davon, welches mindestens einen der folgenden Schritte umfasst
   i) Umsetzung des Oseltamivirs mit Bromcyan in einem organischen Lösungsmittel bei Raumtemperatur
   ii) Umsetzung des in (i) gebildeten Cyanamids mit Hydroxylamin in Dioxan bei Raumtemperatur.

Bevorzugt ist ein Verfahren, wobei das organische Lösungsmittel ein Dichlormethan ist. In einem besonders bevorzugten Aspekt betrifft das erfindungsgemäße Verfahren die Verbindung Amidoxim mit R³= OH gemäß der oben genannten allgemeinen Strukturformel ist.

Als optionaler Schritt kann eine Überführung der Ethylesterfunktion (R⁴= CH₂CH₃, Z = C) in eine Carboxylfunktion (R⁴ = H, Z = C) durch Behandlung mit Hydroxiden in alkoholischen Lösungen erfolgen.

Als Amidierungsreagenz kommen bevorzugt Thioamidiniumsalze in Frage. Bevorzugt wurde das *S*-Naphthylmethylacetamidiniumbromid verwendet.

Als Alkohol kommen bevorzugt organische Verbindungen mit einer gesättigten oder ungesättigten, verzweigten oder nicht verzweigten Kohlenstoffkette der Länge C1 bis C6 in Frage, besonders bevorzugt sind Alkohole, die über eine gesättigte, verzweigte oder unverzweigte Kohlenstoffkette der Länge C1 bis C6 verfügen. Ganz besonders bevorzugt kommen die Alkohole Methanol, Ethanol, Propanol, Butanol, iso-Propanol und tert.-Butanol zum Einsatz. Im Sinne der vorliegenden Erfindung bedeutet alkoholische Lösung, dass hier ein oder mehrere Alkohole entsprechend obiger Definition enthalten sind.

Die Herstellung der erfindungsgemäßen Amidoxime (R³= OH) erfolgt erfindungsgemäß durch ein Verfahren, dass mindestens den folgenden Schritt aufweist: Umsetzung des Oseltamivirs mit einem Hydroximoylchlorid (beispielsweise Acetylhydroximoylchlorid) in organischen Lösungsmitteln (z.B. Dichlormethan) bei Raumtemperatur.

Organische Lösungsmittel sind dem Fachmann bekannt hierzu zählen neben Dichlormethan beispielsweise Chloroform, Aceton, Acetonitril, Methanol, etc.

Darüber hinaus betrifft die vorliegende Erfindung ein Verfahren zur Herstellung einer Verbindung gemäß der vorliegenden Erfindung, umfassend die Umsetzung von Oseltamivir (3*R*,4*R*,5*S*)-4-Acetamido-5-amino-3-(1-ethylpropoxy)cyclohex-1-en-1-carbonsäureethylester oder eines Derivates davon, welches mindestens einen der folgenden Schritte umfasst (i) Umsetzung des Oseltamivirs mit Bromcyan in einem organischen Lösungsmittel bei Raumtemperatur, (ii) Umsetzung des in i gebildeten Cyanamids mit Hydroxylamin in Dioxan bei Raumtemperatur.

Wie bereits vorstehend erläutert, können die anhand von Oseltamivir illustrierten erfindungsgemäß durchgeführten Modifikationen und dementsprechend das erfindungsgemäße Verfahren auch auf Derivate des Oseltamivirs angewendet werden, die beispielsweise an den vorstehend diskutierten Resten modifiziert sind wie in dem Stand der Technik bereits beschriebene Oseltamivir-Derivate, die dementsprechend vom Fachmann zweifelsfrei als äquivalente Ausführungsform der vorliegenden, in den Beispielen illustrierten Erfindung erkannt werden.

Für die Darstellung der erfindungsmäßigen Neuraminidase-Hemmstoffe wurden verschiedene synthetische Strategien verfolgt, die in ihrer Gesamtheit eine Vielzahl an sehr unterschiedlich substituierten Oseltamivir-Abkömmlingen zugänglich machen. Beispiele für verschiedene Synthesewege, die zu unterschiedlichen erfindungsmäßigen Wirkstoffen führen, sind in der folgenden Übersicht dargestellt.

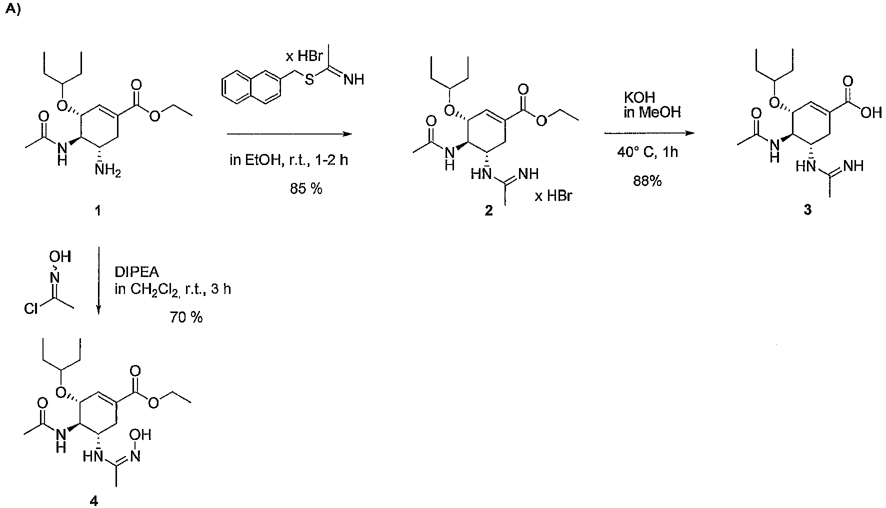

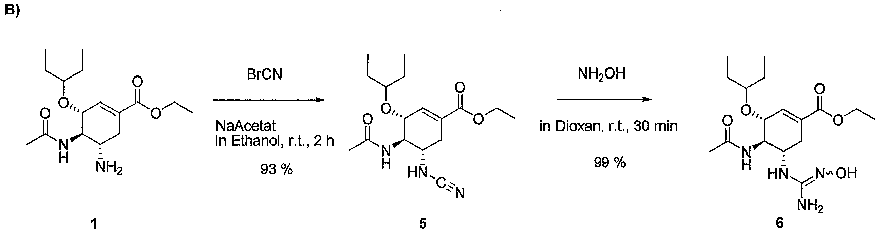

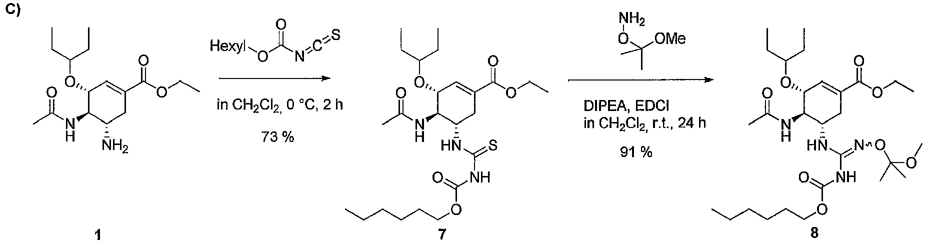

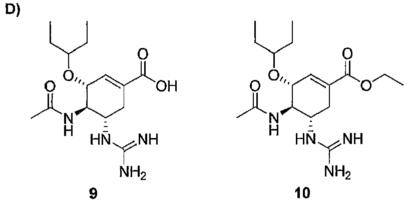

*Übersicht zur Synthese der Neuraminidase-Hemmstoffe. A) Syntheseweg zum Amidin-basierten Oseltamivir-Derivat **3,** sowie zu dem entsprechenden Amidoxim **4;** B) Syntheseweg zum Hydroxyguanidin-basierten Oseltamivir-Derivat **6;** C) Syntheseweg zu weiteren Hydroxyguanidin-basierten Oseltamivir-Derivaten (**8**); D) Strukturformeln von Oseltamivir-Guanidin **9** und seines Ester-Prodrugs **10.***

Für die Darstellung der Amidine (**2, 3**) wurde als Amidierungsreagenz *S*-Naphthylmethylacetamidiniumbromid verwendet (siehe A). Der Einsatz anderer Reagenzien zur Herstellung der Amidine ist ebenfalls möglich. Durch vorangehende *N*-Substitution des Oseltamivirs sowie durch Wahl eines anderen Thioamidiniumsalzes sind auf diesem Weg eine Vielzahl unterschiedlicher Amidine zugänglich. Auch andere bekannte Methoden zum Aufbau einer Amidin-Funktion können hier Verwendung finden. Das Amidoxim **4** wurde bevorzugt durch Verwendung von Acethydroximoylchlorid dargestellt.

Die Synthese der Guanidin-basierten Verbindungen ist unter B) dargestellt. Zwei verschiedene Konzepte zum Aufbau der substituierten und unsubstituierten Hydroxyguanidine wurden dabei umgesetzt. So ist über die Darstellung des Cyanamids **5** das unsubstituierte Hydroxyguanidin **6** zugänglich. Und sehr unterschiedlich *O*,*N*-substituierte Hydroxyguanidine (**8**) sind über carbamoylsubstituierte Thioharnstoffe vom Typ 7 darstellbar (siehe C).

Das Oseltamivir-Guanidin **9** und sein Ester-Prodrug **10** sind ebenfalls dargestellt. Die Darstellung erfolgte nach Shie *et al.* [Shie, J.; Fang, J.; Wang, S.; Tsai, K.; Shyun, Y.; Cheng, E.; Yang, A.; Hsiao, S.; Su, C.; Wong, C., Journal of American Chemical Society 2007, 129, 11892-93].

Es hat sich in den erfindungsgemäß durchgeführten Experimenten gezeigt, dass der Austausch der Amin-Funktion durch eine Amidin-Gruppe vorhandene Influenza-Resistenzmechanismen aushebeln vermag. Die durch durchgeführte Experimente erhaltenen Testdaten aus verschiedenen antiviralen Tests zeigen, dass die Amidin- und Guanidin-Derivate gegenüber Influenza A(H1N1)-Stämmen vergleichbar potent sind wie Oseltamivir und Zanamivir, jedoch zusätzlich wirksam gegen einen Oseltamivir-resistenten Influenza A(H1N1)-Stamm sind. Sie besitzen ferner Wirksamkeit gegenüber verschiedenen Influenza A(H3N2)-Stämmen. Darüber hinaus zeigen die erfindungsgemäßen Verbindungen überraschenderweise eine geringe Toxizität in Zytotoxizitätstests, gute Löslichkeit, gute Aktivierung in die Wirkform sowie eine sehr gute Stabilität unter physiologischen Bedingungen.

Tabelle 1 zeigt die Wirkung der erfindungsgemäßen Substanz 3 und der Substanz 9 im Neuraminidase (NA)-Hemmtest im Vergleich zum Zanamivir und Oseltamivir. Eine genaue Beschreibung des Testes findet sich bei der Beschreibung der Methoden.

**Tabelle 1: Wirkung der Prüf- und Kontrollsubstanzen im NA-Hemmtest**

| **H3N2-Subtyp** | | | | |
|---|---|---|---|---|
| Substanz | 50%ige Hemmkonzentration (nM) gegenüber | | | |
| | Hongkong/8/68 | Sachsen/6/02 | Berlin/10/04 | Rheinl.Pfalz/3911/03 |
| Amidin-Wirkform **3** | 1.0 | 1.2 | 1.3 | 0.7 |
| Guanidin-Wirkform **9** | 1.1 | 1.3 | 1.0 | 0.5 |
| Zanamivir | 0.3 | 0.4 | 0.4 | 0.2 |
| Oseltamivir | 0.2 | 0.1 | 0.1 | 0.1 |

| **H1N1-Subtyp** | | | | |
|---|---|---|---|---|
| Substanz | 50%ige Hemmkonzentration (nM) gegenüber | | | |
| | A/Jena/5258/2009 | A/Jena/5555/09 | A/HH/1580/09 | A/342/2009 |
| Amidin-Wirkform **3** | 0.1 | 0.2 | 0.1 | 11.6 |
| Guanidin-Wirkform **9** | 0.1 | 0.3 | 0.1 | 2.6 |
| Zanamivir | 0.1 | - | - | 0.2 |
| Oseltamivir | 0.1 | 0.1 | 0.1 | resistent |

Die Werte in Tabelle 1 zeigen, dass sowohl **3** als auch **9** im nanomolaren Konzentrationsbereich die NA-Aktivität der acht getesteten Influenza-A-Viren hemmen. Die ermittelten IC50-Werte im NA-Hemmtest waren ungefähr vergleichbar mit denen von Zanamivir und Oseltamivir.

Im Unterschied zu Oseltamivir wirken **3** und **9** auch gegen das Oseltamivir-resistente Isolat A/342/2009. Die für dieses Virus bestimmten 50%igen Hemmkonzentrationen lagen im Vergleich zu Zanamivir 10- (**9**) bzw. 50-fach (**3**) höher.

Die antivirale Wirkung zeigt sich auch im Virusertrag-Hemmtest. Die Ergebnisse des Testes sind in Tabelle 2 dargelegt. Die Amidin-Wirkform (**3**) und die Guanidin-Wirkform (**9**) reduzierten den Titer von Influenzavirus A/Jena/5258/2009 im nanomolaren Konzentrationsbereich zu 90 bzw. 95 %. Eine genaue Beschreibung der Bedingungen des Testes findet sich bei der Beschreibung der Methoden.

**Tabelle 2: Antivirale Wirkung der Testsubstanzen im Virusertrag-Hemmtest mit Influenza-A-Virus in MDCK-Zellen (Madin Darby canine kidney)**

| Substanz | 90- bzw. 95%ige* Hemmkonzentration (µM) gegenüber | |
|---|---|---|
| | A/Jena/5258/2009 | A/342/2009 |
| Amidin-Wirkform **3** | 0.001 (0.011) | 16.0 (22.3) |
| Guanidin-Wirkform **9** | 0.002 (0.003) | 3.0 (14.2) |
| Zanamivir | 0.125 (0.219) | 1.5 (2.5) |
| Oseltamivir | 0.016 (0.021) | resistent |

| | | |
|---|---|---|
| * In Klammern | | |

Auch im zpE-Hemmtest zeigt sich die antivirale Wirkung der erfindungsgemäßen Verbindungen.

Die Replikation der im Test verwendeten Viren führt durch einen stark ausgeprägten zytopathischen Effekt (zpE) zur kompletten Zerstörung der Wirtszellen. Durch die Zugabe antiviral wirksamer Substanzen (100 µl/well; 3 Parallelen/Konzentration, Verdünnungsfaktor 2) lässt sich der Virusinduzierte zpE gezielt hemmen. Im Test wurden unbehandelte und Substanz-behandelte geschlossene Zellrasen mit einer Virusdosis beimpft, die 48 h nach Infektion zu einem vollständigen zpE in den unbehandelten Viruskontrollen führt.

**Tabelle 3: Antivirale Wirkung der Testsubstanzen im zpE-Hemmtest mit Influenza-A-Virus in MDCK-Zellen**

| Substanz | 50%ige Hemmkonzentration (µM) gegenüber | |
|---|---|---|
| | A/Jena/5258/2009 | A/342/2009 |
| Amidin-Wirkform **3** | 2.0 | 12.1 |
| Guanidin-Wirkform **9** | 1.1 | 4.1 |
| Zanamivir | > 19.5 | 0.6 |
| Oseltamivir | 13.2 | resistent |

Die Amidin-Wirkform **3** und die Guanidin-Wirkform **9** hemmten im nicht zytotoxischen Konzentrationsbereich den zpE von Influenzavirus A/Jena/5258/2009 bzw. A/342/2009 wobei die 50%ige Hemmkonzentrationen bei 2.0 und 12.2 µM (Derivat **3**) bzw. bei 1.1 und 4.1 µM (Derivat **9**) lagen. Eine genaue Beschreibung der Bedingungen des Testes findet sich bei der Beschreibung der Methoden.

Ein weiterer Vorteil der Verbindungen ist ihre geringe Toxizität. So erwiesen sich sowohl die Amidin (**3**) als auch die Guanidin-Wirkform (**9**) im getesteten Konzentrationsbereich von 6.25 bis 200 µg/ml als nicht toxisch im Zytotoxizitätstest zur Bestimmung der 50% zytotoxischen Dosis (CC₅₀) der Prüfsubstanzen in MDCK (Madin darby canine kidney)-Zellrasen. Eine genaue Beschreibung der Testbedingungen findet sich bei der Beschreibung der Methoden.

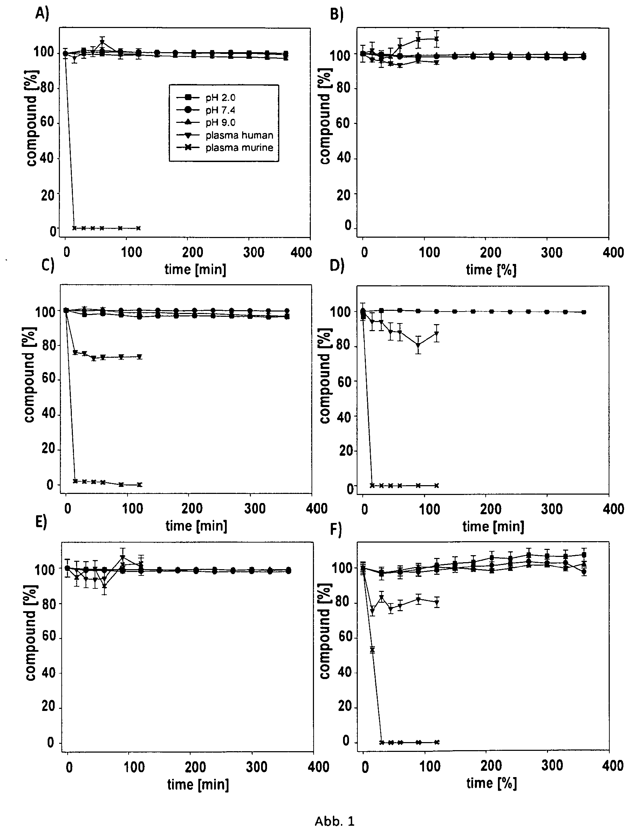

Eine weitere positive Eigenschaft der Verbindungen ist ihre gute Stabilität insbesondere unter physiologischen Bedingungen. Die Untersuchungen zeigten, dass repräsentative Verbindungen sehr stabil im pH-Bereich zwischen 2 und 9 sind (Abbildung 1). Bei allen Verbindungen konnte keinerlei Zersetzung der Verbindung innerhalb des Untersuchungszeitraumes von 6h beobachtet werden.

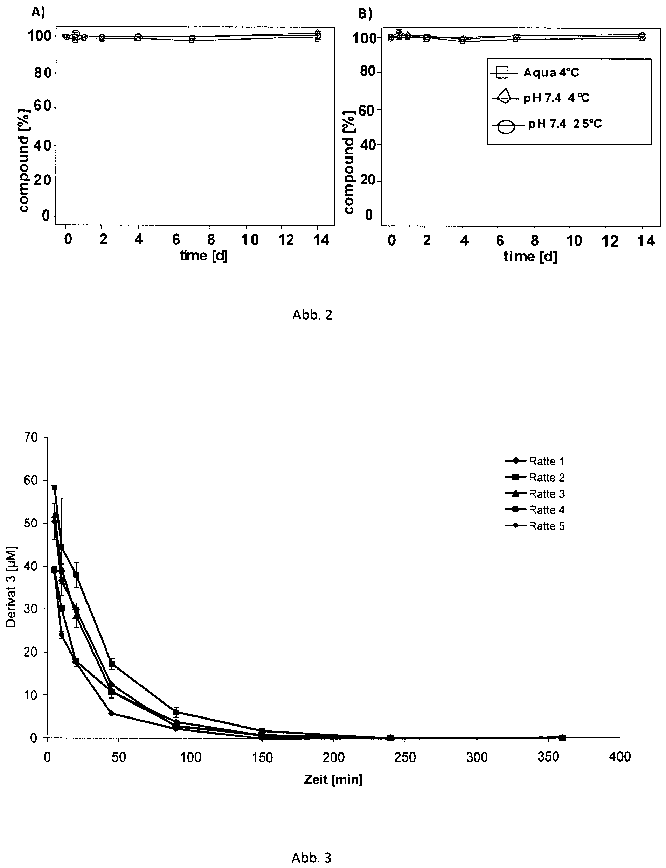

Für die Wirkformen **3** und **9** wurde zusätzlich eine Stabilitätsuntersuchungen über 14 Tage durchgeführt, die zeigte, dass beide Substanzen bei physiologischen pH-Wert sowohl bei 4°C als auch bei RT über den untersuchten Zeitraum stabil sind (Abbildung 2). Die Lagerstabilität in gelöster Form wurde dabei in einer Konzentration von 0,2 mg/ml bestimmt. Dazu wurde die Verbindung in 50 mM KH₂PO₄-Puffer pH 7,4 bzw. Aqua Bidest angelöst und über den Untersuchungszeitraum bei RT (pH 7.4) oder im Kühlschrank bei 4°C (pH 7.4 bzw. Aqua Bidest) gelagert. Es wurde die Konzentration der Wirkform nach 12h, 1d, 2d, 4d, 7d und 14d mit Hilfe der HPLC bestimmt.

Weitere Untersuchungen mit murinem und humanem Plasma zeigten, dass die Esterfunktionen (bei allen Prodrugs die Ethylester der Carboxyfunktion) rasch durch Plasmaenzyme gespalten werden. Die Esterspaltung ist ein wichtiger Schritt der Bioaktivierung, der mit Hilfe dieser Inkubationen nachgewiesen werden konnte. Die Wirkformen **3** und **9** werden durch Plasmaenzyme nicht metabolisiert.

Ein besonderer Vorteil der erfindungsgemäßen Verbindungen ist ihre gute Löslichkeit. Die meisten Verbindungen (**2, 3, 6** und 10*) sind bei allen untersuchten pH-Werten in einer Konzentration >50 mM löslich (Tabelle 4). Zusätzlich zeigte sich, dass alle Substanzen bei einem pH-Wert von 2 besser als 50 mM löslich sind. Somit ist anzunehmen, dass sich alle Verbindungen im sauren Milieu des Magens sehr gut lösen werden. Darüber hinaus sind aber auch die schlechter löslichen Substanzen (**4,** 9 : Referenzverbindung) bei allen pH-Werten noch immer besser als 20 mM löslich. Somit zeigen alle Verbindungen sehr gute Löslichkeitseigenschaften, was in Hinblick auf eine spätere Anwendung als Arzneistoff positiv zu bewerten ist. So sind beispielsweise neben einer oralen Dareichungsform auch flüssige Dareichungsformen (Injektionen, Infusion), welche in der Notfallmedikation benötigt werden, denkbar.

**Tabelle 4: Löslichkeit der untersuchten Verbindungen bei unterschiedlichen pH-Werten.**

| **Verbindung** | **pH-Wert** | **maximal lösliche Konzentration** | |
|---|---|---|---|
| **2** | | **[mg/ml]** | **[mM]** |
| | 9.0 | > 17.7 | > 50 |
| | 7.4 | > 17.7 | > 50 |
| | 2.0 | > 17.7 | > 50 |

| **3** | | | |
|---|---|---|---|
| | 9.0 | > 16.3 | > 50 |
| | 7.4 | > 16.3 | > 50 |
| | 2.0 | > 16.3 | > 50 |

| **4** | | | |
|---|---|---|---|
| | 9.0 | 8.6 ± 0.7 | 23.3 ± 1.8 |
| | 7.4 | 8.6 ± 0.4 | 23.2 ± 1.2 |
| | 2.0 | > 18.5 | > 50 |

| **6** | | | |
|---|---|---|---|
| | 9.0 | > 18.5 | > 50 |
| | 7.4 | > 18.5 | > 50 |
| | 2.0 | > 18.5 | > 50 |

| **9** | | | |
|---|---|---|---|
| | 9.0 | 8.0 ± 0.1 | 24.6 ± 0.2 |
| | 7.4 | 8.6 ± 0.3 | 26.2 ± 1.1 |
| | 2.0 | > 16.3 | > 50 |

| **10** | | | |
|---|---|---|---|
| | 9.0 | > 23.4 | > 50 |
| | 7.4 | > 23.4 | > 50 |
| | 2.0 | > 23.4 | > 50 |

Ein weiterer Vorteil der erfindungsgemäßen Verbindungen ist die Tatsache, dass die Verbindungen nur sehr moderate Plasmaproteinbindungen ausbilden. Die durchgeführten Untersuchungen zeigten, dass alle getesteten Verbindungen Proteinbindungen von unter 40% besitzen (Tabelle 5). Das Risiko einer klinisch relevanten Arzneimittelinteraktion steigt erst ab Proteinbindungen größer als 90%, so dass klinisch relevante Interaktionen durch Proteinbindungen für die hier entwickelten Prodrugs sowie für die Wirkformen nicht zu erwarten sind.

**Tabelle 5: Ermittelte Proteinbindungen in 4%iger Albuminlösung für die untersuchten Verbindungen. Die dargestellten Werte stellen die Mittelwerte aus den Bestimmungen bei drei verschiedenen Konzentrationen dar.**

| **Verbindung** | **Proteinbindung [%]** |
|---|---|
| **3** | 2.2 ± 1.5 |
| **9** | 0.4 ± 2.8 |
| **2** | 34.8 ± 8.8 |
| **10** | 30.1 ± 2.3 |
| **4** | 26.7 ± 2.5 |
| **6** | 37.5 ± 10.1 |

Zusätzlich wurde die Proteinbindung der beiden Wirkformen Oseltamivir-Amidin (**3**) und Oseltamivir-Guanidin (**9**) in humanem Plasma untersucht. Dazu wurde anstelle der 4%igen Albumin-Lösung humanes Plasma verwendet. Es wurden Proteinbindung von 3.7 ± 1.4% für Verbindung **3** und 8.6 ± 3.0% für Verbindung **9** ermittelt. Die Werte liegen erwartungsgemäß etwas über den mit der 4%igen-Albumin-Lösung erhaltenen Werten und sind auf das Vorhandensein weiterer Plasmaproteine (z.B. α₁-saures Glykoprotein) neben dem Albumin zurückzuführen.

Ein weiterer besonderer Vorteil der erfindungsgemäßen Verbindungen ist die Tatsache, dass hier bereits etablierte Prodrug-Konzepte verwendet werden können. Die Carbonsäure wird in üblicher Form als Ester eingesetzt. Für die Amidin- und Guanidinfunktion wurde das mittlerweile ebenfalls etablierte *N*-Hydroxy-Konzept verwendet. Auch unter dem Aspekt einer retardierten Freisetzung der Wirkform nach oraler oder parenteraler Applikation, sind die beschriebenen Prodrug-Formen interessant.

Für die Prodrugs wurden ebenfalls *in vitro* Untersuchungen zu Stabilität, Löslichkeit und Aktivierung in die Wirkform unternommen. Die Ergebnisse zeigten, dass die Verbindungen eine ausreichende Stabilität, eine sehr gute Löslichkeit besitzen und dass die Aktivierung in die Wirkform durch verschiedene Enzympräparationen in gutem Umfang stattfindet.

Die Aktivierung der Prodrugs in ihre Wirkformen wurde *in vitro* mittels subzellulärer Enzympräparationen bestimmt. Als Enzympräparationen wurden 9000g Überstände, Mikrosomen und Mitochondrien aus humanen und porcinen Lebergeweben verwendet. Die Inkubationsansätze setzten sich aus 500 µM Prodrug, 500 µM NADH, 1 U Esterase und 0.3 mg Enzympräparation, gelöst in 150 µl 100 mM Phosphatpuffer pH 6.3, zusammen. Die Inkubation erfolgte über 30 min bei 37°C im Schüttelwasserbad. Beendet wurde die Inkubation durch Zugabe von 150 µl Acetonitril. Anschließend wurden die Proben 10 min geschüttelt und das ausgefällte Protein bei 10.000 RPM für 15 min abzentrifugiert. Der Überstand wurde mit Hilfe der HPLC vermessen.

Die ermittelten Umsetzungsraten sind in Tabelle 6 aufgeführt.

Die durchgeführten *in vitro* Aktivierungsstudien zeigten, dass alle entwickelten Prodrugs in die aktive Form **3** bzw. **9** überführt werden (Tabelle 4). Das heißt, sowohl die Esterspaltung läuft ab, wie auch schon bei den Stabilitätsprüfungen in humanem bzw. murinem Plasma nachgewiesen werden konnte, und zusätzlich konnte in diesen Inkubationen die Reduktion des Amidoxims bzw. des *N*-OH-Guanidins detektiert werden. Zusammenfassend kann festgehalten werden, dass es sich bei den Verbindungen **2, 4, 6** und 10 : Referenzverbindung um geeignete Prodrugs der Wirkformen **3** und **9** handelt. Diese Studie erweist nur den generellen Nachweis, dass die Bioaktivierung der Verbindungen abläuft. Die Umsetzungsraten sollten *in vivo* deutlich höher liegen.

**Tabelle 6: Aktivierung des Prodrugs in die Wirkform mit subzellularen Enzympräparationen**

| | **Umsetzungsrate [nmol/min/mg Protein]** | | | |
|---|---|---|---|---|
| **Enzympräparation** | **2** | **4** | **8** | **10** |
| Humane Leber Mitochondrien | 0.56 ± 0.04 | 0.22 ± 0.16 | 1.74 ± 0.06 | 0.79 ± 0.04 |
| Schweine Leber Mitochondrien | 0.15 ± 0.01 | 0.51 ± 0.06 | 0.67 ± 0.02 | 0.21 ± 0.01 |
| Humane Leber 9000g Überstand | 0.20 ± 0.02 | 0.56 ± 0.06 | 0.76 ± 0.09 | 0.32 ± 0.03 |
| Schweine Leber 9000g Überstand | 0.42 ± 0.10 | 5.34 ± 0.15 | 1.53 ± 0.14 | 0.22 ± 0.01 |
| Humane Leber Mikrosomen | 0.64 ± 0.01 | 2.30 ± 0.20 | 1.76 ± 0.05 | 1.06 ± 0.02 |
| Schweine Leber Mikrosomen | 0.30 ± 0.11 | 7.51 ± 0.66 | 4.77 ± 0.13 | 0.22 ± 0.01 |

Die enzymatische Hydrolyse des Carbonsäure-Ethylesters wurde an den Prodrugs **2** und **10** näher untersucht. Als Enzymquelle wurden hierzu unspezifische Carboxylesterasen aus Schweinleber verwendet. Die Inkubationsansätze enthielten 200 µM Prodrug und 3 U Esterase gelöst in 200 µl 100 mM Phosphatpuffer pH 7.4. Die Inkubation erfolgte über einen Zeitraum von 60 min bei 37°C. Alle 15 min wurden Proben mit Hilfe der HPLC analysiert.

Die Inkubationen zeigten, dass beide Prodrugs durch die Esterase zur jeweiligen Wirkform aktiviert werden. Die ermittelten Umsetzungsraten betrugen 0.83 ± 0.14 nmol / min / mg Protein (Prodrug **2**) und 1.35 ± 0.15 nmol / min / mg Protein (Prodrug **10**).

Ein besonderer Vorteil der erfindungsgemäßen Verbindungen ist ihre gute Bioverfügbarkeit.

Die neu-entwickelten Neuraminidase Inhibitoren wurden in einer Tierstudie an Ratten hinsichtlich ihrer oralen Bioverfügbarkeit untersucht. Alle getesteten Verbindungen zeigen dabei, dass sie sowohl aus dem Magen-Darm-Trakt aufgenommen als auch in ihre Wirkform metabolisiert werden. Die Metabolisierung der Neuraminidase Inhibitoren **4** und **6** ist zur Verdeutlichung dargestellt.

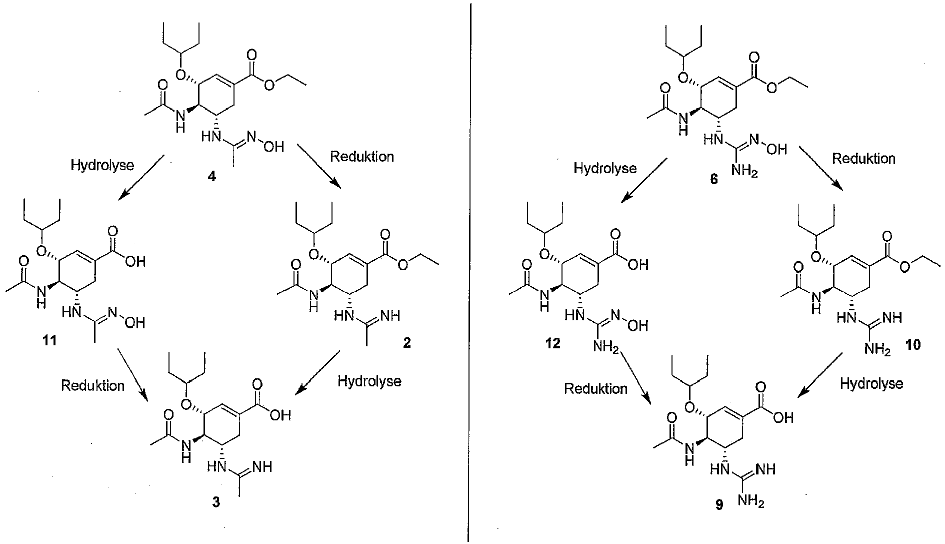

### Bioverfügbarkeit der Oseltamivir-Amidoxim-Derivate (2, 3 und 4)

Die höchsten Plasmaspiegel wurden nach Applikation des Osteltamivir-Amidoxim-Derivats **4** erhalten. Nach oraler Applikation wurden Plasmaspiegel der Wirkform **3** im µmolaren Bereich über den gesamten untersuchten Zeitraum von 6 h bestimmt. Zudem konnte nach Applikation von Derivat **4** in geringerem Ausmaß auch der Intermediär-Metabolit **11** nachgewiesen werden, welcher in die Wirkform **3** metabolisiert wird. Die Bioverfügbarkeit des Amidoxim-Derivats **4** wurde mit 31.3 % bestimmt; die Plasma-Halbwertszeit von **3** beträgt ca. 112 min (Tabelle 2). Der einzige derzeit zugelassene Neuraminidase Inhibitor mit oraler Bioverfügbarkeit ist das Oseltamivir (Tamiflu®). Tierstudien an Ratten zeigten für diese Verbindung eine orale Bioverfügbarkeit von 36% und eine Plasma-Halbwertszeit von 44 min. [E. J. Eisenberg, A. Bidgood, K. C. Cundy, Antimicrob Agents Chemother 1997, 41(9), 1949-1952].

Die erfindungsgemäß neu-entwickelte Verbindung **4** ist demnach hinsichtlich Bioverfügbarkeit vergleichbar. Die entscheidenden Vorteile dieser Verbindung sind zum einem die verlängerte Halbwertszeit, welche langwirksame Plasmaspiegel ermöglicht; zum anderen die deutliche Überlegenheit bei der Wirksamkeit gegenüber Oseltamivir-resistenten Influenza-Stämmen.

Die Applikation der anderen Verbindungen (**2, 3**) lieferte ebenfalls detektierbare Plasmaspiegel von Derivat **3,** die jedoch niedriger ausfielen als nach Applikation von **4.**

Die Ergebnisse der intravenösen Applikation von Derivat **3** sind in der folgenden Tabelle sowie der Abbildung 3 dargestellt.

Mittelwerte aller Plasmaspiegel nach intravenöser Applikation von Derivat **3**

| **Zeit** | **Konz. [µM]** | **Stabw. [µM]** |
|---|---|---|
| 5 | 47.78 | 8.57 |
| 10 | 34.91 | 8.06 |
| 20 | 26.40 | 8.65 |
| 45 | 11.40 | 4.16 |
| 90 | 3.49 | 1.55 |
| 150 | 1.02 | 0.52 |
| 240 | 0.11 | 0.07 |
| 360 | 0.01 | 0.01 |

Die orale Applikation von Derivat **4** lieferte die folgenden Plasmaspiegel von Derivat **3**
Mittelwerte aller Plasmaspiegel von Derivat **3** nach oraler Applikation von Derivat **4**

| **Zeit [min]** | **Konz. [µM]** | **Stabw. [µM]** |
|---|---|---|
| 0 | 0.00 | 0.00 |
| 30 | 6.50 | 1.90 |
| 60 | 8.98 | 2.53 |
| 90 | 8.80 | 2.30 |
| 120 | 8.10 | 1.60 |
| 180 | 6.43 | 1.50 |
| 240 | 5.34 | 1.50 |
| 360 | 2.19 | 1.08 |

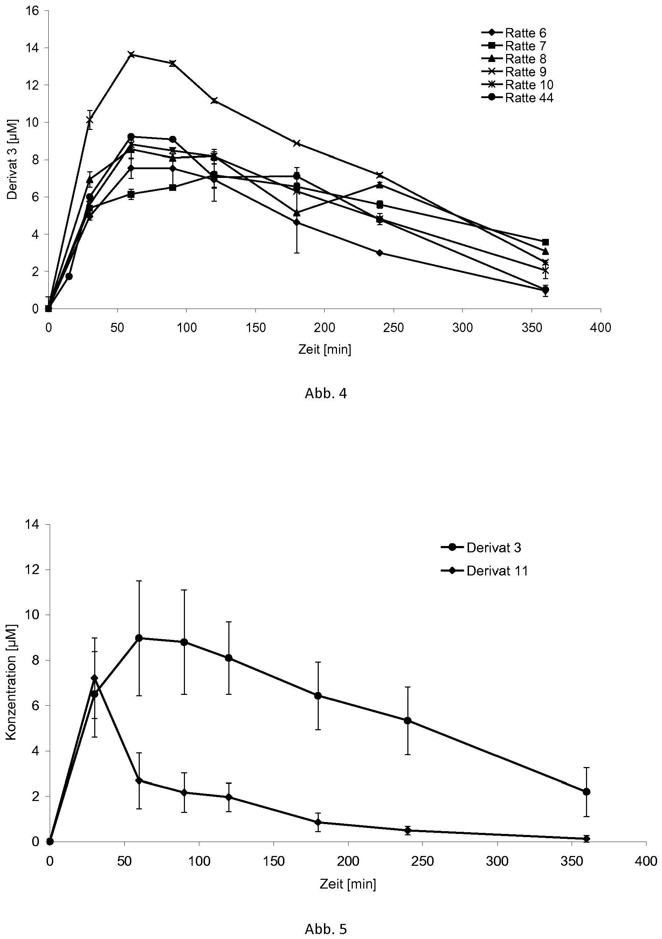

Abbildung 4 zeigt die Ergebnisse graphisch.

Nach oraler Applikation von Derivat **4** ist Derivat **11** als Metabolit nachweisbar. Dieses ist auch aus Abbildung 5 zu erkennen.

Mittelwerte aller Plasmaspiegel von Intermediär-Metabolit **11** nach oraler Applikation von Derivat **4**

| **Zeit [min]** | **Konz. [µM]** | **Stabw. [µM]** |
|---|---|---|
| 0 | 0.00 | 0.00 |
| 30 | 7.22 | 1.77 |
| 60 | 2.68 | 1.23 |
| 90 | 2.16 | 0.86 |
| 120 | 1.96 | 0.63 |
| 180 | 0.85 | 0.41 |
| 240 | 0.49 | 0.18 |
| 360 | 0.13 | 0.15 |

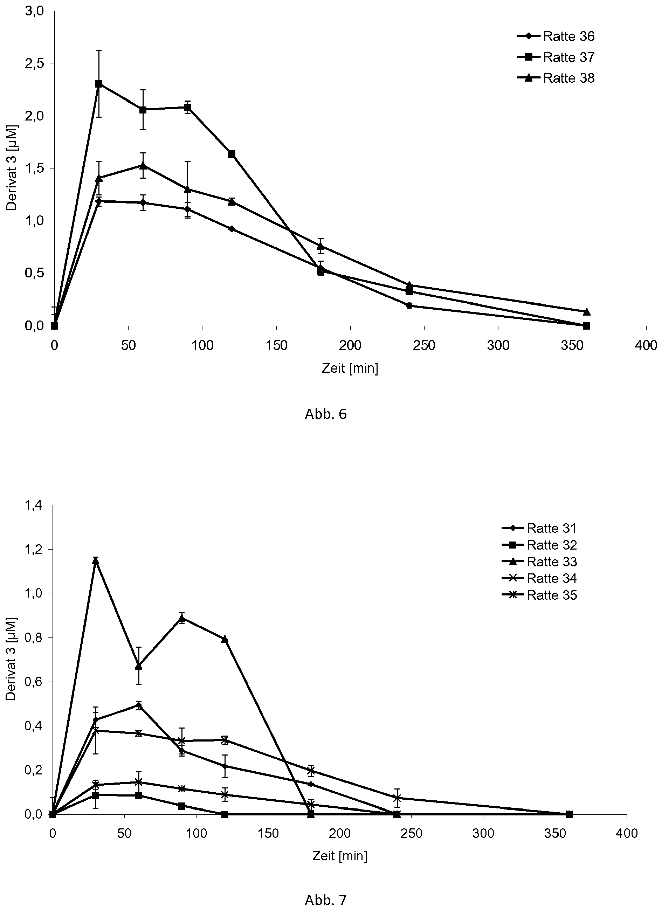

Die orale Applikation von Derivat **3** führt zu den folgenden Plasmaspiegeln siehe auch Abbildung 6

Mittelwerte aller Plasmaspiegel von Derivat **3** nach oraler Applikation von Derivat **3**

| **Zeit [min]** | **Konz. [µM]** | **Stabw. [µM]** |
|---|---|---|
| 0 | 0.00 | 0.00 |
| 30 | 1.63 | 0.11 |
| 60 | 1.59 | 0.18 |
| 90 | 1.50 | 0.12 |
| 120 | 1.25 | 0.11 |
| 180 | 0.61 | 0.04 |
| 240 | 0.30 | 0.03 |
| 360 | 0.04 | 0.00 |

Die orale Applikation von Derivat **2** führt zu den folgenden Plasmaspiegeln siehe auch Abbildung **7**

Mittelwerte aller Plasmaspiegel von Derivat **3** nach oraler Applikation von Derivat **2**

| **Zeit [min]** | **Konz. [µM]** | **Stabw. [µM]** |
|---|---|---|
| 0 | 0 | 0 |
| 30 | 0.43 | 0.21 |
| 60 | 0.35 | 0.12 |
| 90 | 0.33 | 0.17 |
| 120 | 0.29 | 0.16 |
| 180 | 0.07 | 0.04 |
| 240 | 0.01 | 0.01 |
| 360 | 0.00 | 0.00 |

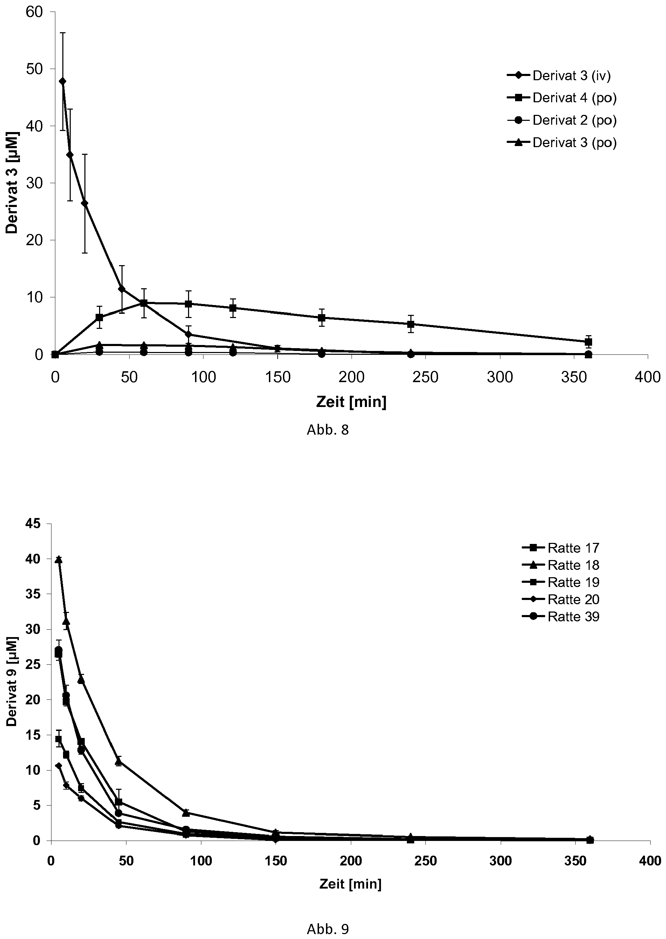

Die Abbildung 8 und die folgende Tabelle zeigt eine Übersicht der Ergebnisse aus der Applikation der Amidin-basierten Neuramidase Inhibitoren (**2,3** und **4**).

**Tabelle 7: Pharmakokinetische Parameter der Amidin-basierten Neuraminidase Inhibitoren**

| **Derivat** | **3** | **3** | **4** | **2** |
|---|---|---|---|---|
| **Applikation** | Iv | Oral | Oral | Oral |
| **Ratten** (n) | 5 | 3 | 6 | 5 |
| **MW** [g/mol] | 325 | 325 | 369 | 434 (HBr) |
| **Dosis** [mg/kg] | 10 | 50 | 50 | 50 |
| **Dosis** (Equi) | 1 | 5 | 4.40 | 3.74 |
| **AUC** | 576.8 | 86.4 | 793.2 | 17.0 |
| **tₘₐₓ** [min] | - | 44.1 | 91.8 | 30.9 |
| **cₘₐₓ** [µg/ml] | - | 0.7 | 2.8 | 0.2 |
| **MRT** [min] | 35.2 | 112.0 | 206.8 | 90.8 |
| **t_{1/2}** [min] | 38.5 | 40.7 | 111.7 | 37.8 |
| **Bioverfügbarkeit** [%] | **100** | **3.0** | **31.3** | **0.8** |

| | | | | |
|---|---|---|---|---|
| AUC = Area under the curve; tₘₐₓ = Zeitpunkt, an dem der maximale Plasmaspiegekl gemessen wurde. cₘₐₓ = maximale Plasmakonzentration, die bestimmt wurde; MRT = Mean Residence Time t_{1/2} = Plasmahalbwertszeit; Die MRT (Mean Residence Time) ist ähnlich wie die Plasmahalbwertszeit t_{1/2} ein Maß für die Verweildauer einer Substanz im Körper. Sie ist eine klassische pharmakokinetische Größe, welche ducrh Division der AUMC (Area under the first moment curve) durch die AUC erhalten wird; | | | | |

### Bioverfügbarkeit der Oseltamivir-Hydroxyguanidin-Derivate (6, 9, 10)

Die Analyse der Plasmaproben lieferte nach Applikation aller untersuchten Derivate detektierbare Plasmaspiegel von Derivat **9** über den Zeitraum von 6 h. Im Vergleich zu den Oseltamivir-Amidoxim-Derivaten (siehe a) fielen die ermittelten Plasmaspiegel jedoch deutlich geringer aus. Die Plasmakonzentrationen von Derivat **9** wurden im dreistelligen nanomolaren Bereich bestimmt und sind somit im Vergleich zu Derivat **4** ca. um den Faktor 10 erniedrigt. Nach Applikation von Derivat **6** konnte auch der Intermediär-Metabolit **12** nachgewiesen werden, welcher in die Wirkform **9** metabolisiert wird.

Die Bioverfügbarkeit des Hydroxyguanidin-Derivats **6** wurde mit 1.7 % bestimmt; die Plasma-Halbwertszeit von **9** beträgt ca. 98 min. Die orale Bioverfügbarkeit der anderen untersuchten Oseltamivir-Derivate auf Guanidin-Basis (**9, 10**) unterscheidet sich nicht signifikant von Derivat **6.**

**Tabelle 8: Pharmakokinetische Parameter der Guanidin-basierten Neuraminidase Inhibitoren**

| **Derivat** | **9** | **9** | **6** | **10** |
|---|---|---|---|---|
| **Applikation** | Iv | Oral | Oral | Oral |
| **Ratten** (n) | 5 | 3 | 4 | 5 |
| **MW** [g/mol] | 326 | 326 | 370 | 468 (TFA) |
| **Dosis** [mg/kg] | 10 | 50 | 50 | 50 |
| **Dosis** (Equi) | 1 | 5 | 4.40 | 3.48 |
| **AUC** | 292.5 | 26.3 | 21.9 | 11.5 |
| **tₘₐₓ** [min] | - | 120.0 | 112.1 | 61.3 |
| **cₘₐₓ** [µg/ml] | - | 0.3 | 0.1 | 0.1 |
| **MRT** [min] | 54.1 | 129.2 | 205.7 | 124.8 |
| **t_{1/2}** [min] | 120.0 | 186.3 | 97.7 | 50.0 |
| **Bioverfügbarkeit** [%] | **100** | **1.8** | **1.7** | **1.1** |

Die Ergebnisse der intravenösen Applikation von Derivat **9** sind in der folgenden Tabelle sowie der Abbildung 9 dargestellt.
Mittelwerte aller Plasmaspiegel nach intravenöser Applikation von Derivat **9**

| **Zeit** | **Konz. [µM]** | **Stabw. [µM]** |
|---|---|---|
| 5 | 23.71 | 11.61 |
| 10 | 18.30 | 8.94 |
| 20 | 12.67 | 6.70 |
| 45 | 5.05 | 3.73 |
| 90 | 1.67 | 1.30 |
| 150 | 0.54 | 0.36 |
| 240 | 0.23 | 0.16 |
| 360 | 0.11 | 0.06 |

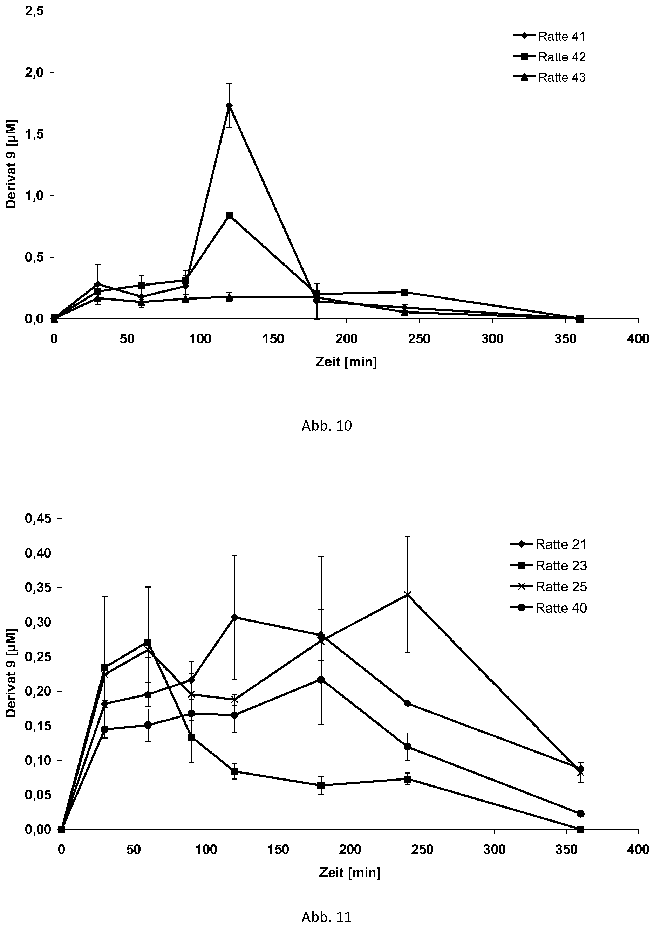

Die orale Applikation von Derivat **9** führt zu den folgenden Plasmaspiegeln siehe auch Abbildung **10**
Mittelwerte aller Plasmaspiegel von Derivat **9** nach oraler Applikation von Derivat **9**

| **Zeit [min]** | **Konz. [µM]** | **Stabw. [µM]** |
|---|---|---|
| 0 | 0.00 | 0.00 |
| 30 | 0.22 | 0.06 |
| 60 | 0.19 | 0.07 |
| 90 | 0.24 | 0.08 |
| 120 | 0.91 | 0.78 |
| 180 | 0.17 | 0.03 |
| 240 | 0.12 | 0.09 |
| 360 | 0.00 | 0.00 |

Die orale Applikation von Derivat **6** führt zu den folgenden Plasmaspiegeln siehe auch Abbildung 11
Mittelwerte aller Plasmaspiegel von Derivat **9** nach oraler Applikation von Derivat **6**

| **Zeit [min]** | **Konz. [µM]** | **Stabw. [µM]** |
|---|---|---|
| 0 | 0.00 | 0.00 |
| 30 | 0.20 | 0.04 |
| 60 | 0.22 | 0.06 |
| 90 | 0.18 | 0.04 |
| 120 | 0.19 | 0.08 |
| 180 | 0.21 | 0.03 |
| 240 | 0.18 | 0.11 |
| 360 | 0.05 | 0.04 |

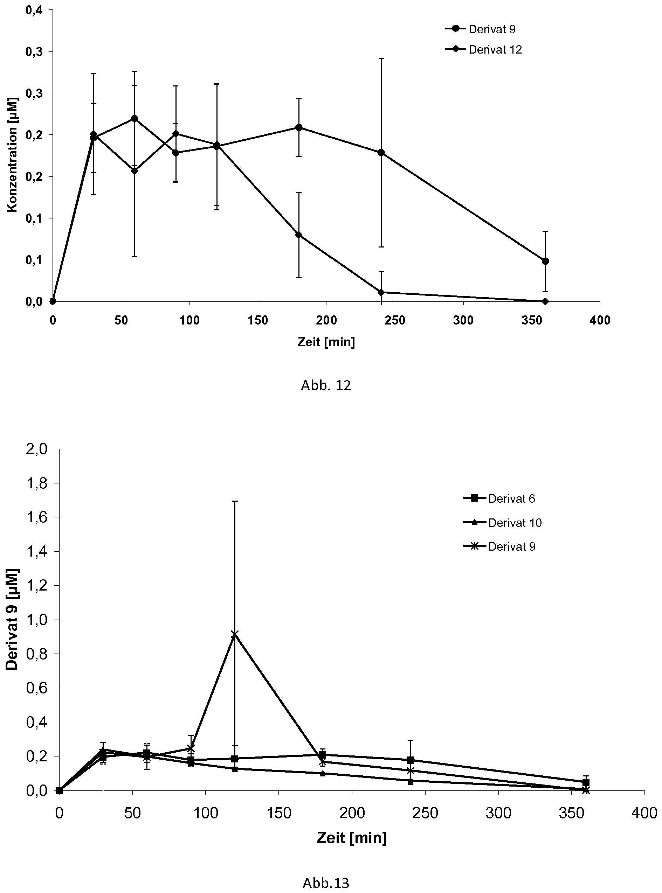

Nach oraler Applikation von Derivat **6** ist Derivat **12** als Metabolit nachweisbar. Dieses ist auch aus Abbildung 12 zu erkennen.

Mittelwerte aller Plasmaspiegel von Intermediär-Metabolit **12** nach oraler Applikation von Derivat **6**

| **Zeit [min]** | **Konz. [µM]** | **Stabw. [µM]** |
|---|---|---|
| 0 | 0.00 | 0.00 |
| 30 | 0.20 | 0.07 |
| 60 | 0.16 | 0.10 |
| 90 | 0.20 | 0.06 |
| 120 | 0.19 | 0.07 |
| 180 | 0.08 | 0.05 |
| 240 | 0.01 | 0.03 |
| 360 | 0.00 | 0.00 |

Abbildung **13** zeigt eine Übersicht der Ergebnisse aus der Applikation der Guanidin-basierten Neuramidase Inhibitoren (**6, 9, 10**).

### Material und Methoden: Ausführungsbeispiele

### Synthesen (3R,4R,5S)-4-Acetamido-5-(N-acetimidamido)-3-(1-ethylpropoxy)cyclohex-1-en-1-carbonsäure-ethylester Hydrobromid (2)

Es werden 1 g Oseltamivir (3.2 mmol) in 10 mL Ethanol gelöst und der Ansatz auf 0 °C gekühlt. Zu dieser Lösung werden 1.04 g an *S*-(Naphthylmethyl)acetimidobromid (1.1 Äquivalente) zugegeben und anschließend eine Stunde bei Raumtemperatur gerührt. Der Ansatz wird im Vakuum einkonzentriert und mit ca. 80 mL Wasser aufgenommen. Diese Lösung wird mit wenig Diethylether gewaschen und anschließend im Vakuum einkonzentriert. Das Produkt (85 %) enthält an dieser Stelle noch Kleinstmengen der Ausgangsverbindung, welche nur mittels Säulenchromatographie entfernt (DCM/MeOH, 5-10%) werden konnte. Ausbeute: 960 mg (71 %) eines weißen Feststoffs.
DC: *R_{f} =* 0.65 (DCM/MeOH, 9:1)
¹H-NMR (DMSO-*d*₆, 300 MHz):
δ/ppm = 0.79 (t, ³*J*= 7.4 Hz, 3H), 0.85 (t, ³*J*= 7.4 Hz, 3H), 1.23 (t, ³*J*= 7.1 Hz, 3H), 1.44 (m_{c}, 4H), 1.83 (s, 3H), 2.11 (s, 3H), 2.33 (m_{c}, 1H), 2.67 (dd, ²*J*= 17.6 Hz, ³*J*= 4.7 Hz, 1H), 3.42 (quin, ³*J*= 5.6 Hz, 1H), 3.82 (m_{c}, 1H), 4.05 (m_{c}, 1H), 4.17 (q, ³*J*= 7.1 Hz, 2H), 4.35 (m_{c}, 1H), 6.69 (m_{c}, 1H), 8.04 (br d, ³*J*= 9.0 Hz, 1H), 8.63 (br s, 1H), 9.25, 9.35 (2 x br s, 1H).
MS (ESI): *m*/*z* = 354 [M + H]⁺.

### 3R,4R,5S)-4-Acetamido-5-(N-acetimidamido)-3-(1-ethylpropoxy)cyclohex-1-en-1-carbonsäure (3)

Der Amidin-Ethylester des Oseltamivirs (217 mg, 0.5 mmol) wird in 10 mL MeOH mit 1.5 mL einer 1 M methanolischen KOH (3 Äquivalente) versetzt und 1 Stunde bei 40 °C gerührt bis kein Edukt mehr auf der DC zu detektieren ist. Die Lösung wird mit Wasser verdünnt und der pH Wert wird mit 1 M HCl auf 7-8 eingestellt. Die Lösung wird im Anschluss zur Trockne einkonzentriert und der Rückstand wird mittels Flashchromatographie auf Umkehrphase (RP-18-Säule, Eluent: Wasser, Detektion: Iodkammer) gereinigt. Das Produkt wird nach Lyophilisation als weißes Pulver isoliert.

Ausbeute: 88 % eines feinen, weißen Pulvers.
¹H-NMR (D₂O, 300 MHz):
δ/ppm = 0.84 (t, ³*J*= 7.4 Hz, 3H), 0.89 (t, ³*J*= 7.4 Hz), 3H, 1.38-1.63 (m, 4H), 2.03 (s, 3H), 2.23 (s, 3H), 2.43 (m_{c} 1H), 2.82 (dd, ²*J*= 17.5 Hz, ³*J*= 4.8 Hz, 1H,), 3.53 (quin, ³*J*= 5.4 Hz, 1H), 3.93-4.09 (m, 2H), 4.36 (m_{c}, 1H), 6.71 (br s, 1H).
MS (ESI): *m*/*z* = 348 [M + Na]⁺, 326 [M + H]⁺.
HRMS (ESI): *m*/*z* ber. für C₁₆H₂₇N₃O₄ [M + H]⁺: 326.20743, gef.: 326.20737.

### (3R,4R,5S)-4-Acetainido-5-[N-(N'-hydroxy)acetimidamido]-3-(1-ethylpropoxy)cyclohex-1-en-1-carbonsäureethylester (4)

465 mg Oseltamivir (1.49 mmol), 290 mg DIPEA (389 µL, 1.5 equiv) werden in 5 mL Dichlormethan gelöst und auf 0°C gekühlt. Zu dieser Lösung wird langsam (tropfenweise) frisch hergestelltes Acethydroximoylchlorid (209 mg, 1.5 equiv) zugegeben. Die Mischung wird vier Stunden bei Raumtemperatur gerührt, mit 15 mL Wasser versetzt, eine weitere Stunde gerührt und anschließend im Scheidetrichter getrennt. Die wässrige Phase wird viermal mit Dichlormethan extrahiert, um die Ausbeute am gewünschten Amidoxim zu erhöhen. Die vereinigten organischen Phasen werden über Na₂SO₄ getrocknet und im Vakuum einkonzentriert. Das Rohprodukt wird mittels Säulenchromatographie auf Kieselgel gereinigt (DCM/MeOH, 9:1).
Ausbeute: 70 % farbloser, kristalliner Feststoff
DC: *R_{f} =* 0.29 (DCM/MeOH, 9:1)
¹H-NMR (DMSO-*d*₆, 300 MHz):
δ/ppm = 0.80 (t, ³*J*= 7.4 Hz, 3H), 0.85 (t, ³*J*= 7.4 Hz, 3H), 1.23 (t, ³*J*= 7.1 Hz, 3H), 1.43 (m_{c}, 4H), 1.80 (s, 3H), 1.95 (s, 3H), 2.38 (m_{c}, 1H), 2.62 (dd, ²*J*= 17.4 Hz, ³*J*= 5.0 Hz, 1H), 3.40 (quin, ³*J*= 5.6 Hz, 1H), 3.65 (m_{c}, 1H) 3.78 (dd, ²*J*= 17.4 Hz, ³*J*= 8.7 Hz, 1H) 4.15 (q, ³*J*= 7.1 Hz, 2H), 4.19 (m_{c}, 1H), 6.67 (m_{c}, 1H), 6.81 (br d, 1H, ³*J*= 9.1 Hz), 7.99 (d, 1H, ³*J*= 8.6 Hz), 9.73 (br s, 1H).
MS (ESI): *m*/*z* = 392 [M + Na]⁺, 370 [M + H]⁺, 354 [M - OH + H]⁺.
HRMS (ESI): *m*/*z* ber. für C₁₈H₃₁N₃O₅ [M + H]⁺: 370.23365, gef.: 370.23379.

### (3R,4R,5S)-4-Acetamido-5-[-N-(N'hydroxy)guanidino]-3-(1-ethylpropoxy)cyclohex-1-en-1-carbonsäureethylester (6)

213 mg des Cyanamids (0.6 mmol) werden in 5 mL trockenem Dioxan gelöst und genau 1 Äquivalent freies Hydroxylamin (20 mg) zugegeben. Es wird 30 Minuten bei Raumtemperatur gerührt, einkonzentriert und nach mehrmaliger Zugabe und Entfernen von Dichlormethan und Diethylether ein weißer Feststoff erhalten.
Ausbeute: 222 mg (100 %) eines weißen Feststoffs
DC: *R_{f}* = 0.20 (EtOAc/MeOH, 6:4)
¹H-NMR (DMSO-*d*₆, 300 MHz):
δ/ppm = 0.80 (t, ³*J*= 7.3 Hz, 3H), 0.84 (t, ³*J*= 7.4 Hz, 3H), 1.24 (t, ³*J*= 7.1 Hz, 3H), 1.40 (m, 4H), 1.83 (s, 3H), 1.99-2.07 (m, 1H), 2.86 (dd, ²*J*= 16.7 Hz, ³*J*= 2.5 Hz, 1H), 3.38 (quin, ³*J*= 5.5 Hz, 1H), 3.49 (m, 1H), 3.80 (m, 1H), 4.01 (m, 1H), 4.14 (q, ³*J*= 7.1 Hz, 2H), 4.24 (m, 1H), 4.92 (s, 2H), 6.64 (m, 1H), 7.72 (br s, 1H), 7.79 (d, ³*J*= 8.8 Hz, 1H).
MS (ESI):
*m*/*z* = 741 [2M + H]⁺, 393 [M + Na]⁺, 386, 371 [M + H]⁺.
HRMS (ESI): *m*/*z* ber. für C₁₇H₃₀N₄O₅ [M + H]⁺: 371.22890, gef.: 371.22911.

### (3R,4R,5S)-4-Acetatnido-5-[N-(N'-n-hexyloxycarbonyl)thioureido]-3-(1-ethylpropoxy)-cyclohex-1-en-1-carbonsäureethylester (7)

500 mg Oseltamivir (1.6 mmol) werden in 50 mL trockenem Dichlormethan gelöst und äquimolare Mengen an Hexyloxycarbonylisothiocyanat (aus einer etwa 0.5 M Lösung in Dichlormethan) langsam zugetropft. Es wird 2 Stunden bei Raumtemperatur gerührt und anschließend mit 1% HCl, Wasser, NaCl-Lösung gewaschen. Die organische Phase wird über Na₂SO₄ getrocknet und am Rotationsverdampfer eingeengt. Das Rohprodukt kann mit Cyclohexan verrieben, bzw. gewaschen werden und ist dadurch ausreichend rein für die nächste Umsetzung. Für die Elementaranalyse wurde die Verbindung mittels Säulenchromatographie über Kieselgel (Cy/EtOAc, 6:4) weiter gereinigt.
Ausbeute: 600 mg (75%) eines weiß-gelblichen Feststoffs
DC: *R_{f}* = 0.20 (Cy/EtOAc, 6:4)
¹H-NMR (DMSO-*d*₆, 300 MHz):
δ/ppm = 0.79 (t, ³*J*= 7.4 Hz, 3H), 0.84 (t, ³*J*= 7.3 Hz, 3H), 0.87 (t, ³*J*= 6.8 Hz, 3H), 1.23 (t, ³*J*= 7.2 Hz, 3H), 1.30 (m_{c}, 6H), 1.45 (m_{c}, 4H), 1.57 (m_{c}, 2H), 1.80 (s, 3H), 2.30 (dd, 1H, ²*J*= 17.8 Hz, ³*J*= 6.8 Hz), 2.90 (dd, 1H, ²*J*= 17.8 Hz, ³*J*= 5.0 Hz), 3.43 (quin, 1H, ³*J*= 5.4 Hz), 4.07 (m_{c}, 4H), 4.16 (q, 2H, ³*J*= 7.1 Hz), 4.55 (m_{c}, 1H), 6.74 (br s, 1H), 7.91 (br d, 1H, ³*J*= 8.0 Hz), 9.98 (d, 1H, ³*J*= 7.6 Hz), 10.90 (s, 1H).
MS (ESI):
*m*/*z* = 500 [M + H]⁺, 483, 412.

### (3R,4R,5S)-4-Acetamido-5-[N-(N'-n-hexyloxycarbonyl)-(N"-(2-methoxypropan-2yl)oxy)guanidino]-3-(1-ethylpropoxy)cyclohex-1-en-1-carbonsäureethylester (8)

Es werden 358 mg des Oseltamivir-Hexylthiohamstoffs (0.72 mmol) in 10 mL trockenem Dichlormethan gelöst und 151 mg *O*-(2-Methoxypropan-2-yl)hydroxylamin (2 Äquivalente), 251 µL DIPEA (2 Äquivalente), 276 mg EDCI (2 Äquivalente) zugegeben. Der Ansatz wird 1.5 Tage bei Raumtemperatur gerührt, einkonzentriert und mittels Säulenchromatographie über Kieselgel aufgearbeitet (DCM/MeOH, 0-2%).
DC: *R_{f}* = 0.39 (DCM/MeOH, 98:2)
Ausbeute: 374 mg (91%) eines weißen Feststoffs, der bei -20 °C gelagert wird.
¹H-NMR (DMSO-*d*₆, 300 MHz):
δ/ppm = 0.83 (m_{c}, 9H), 1.27 (m_{c}, 12H), 1.27 (m_{c}, 12H), 1.45 (m_{c}, 4H), 1.57 (m_{c}, 2H), 1.80 (s, 3H), 2.30 (dd, 1H, ²*J*= 18.1 Hz, ³*J*= 7.1 Hz), 2.90 (dd, 1H, ²*J*= 18.1 Hz, ³*J*= 5.2 Hz), 3.06 (s, 3H), 3.43 (quin, 1H, ³*J*= 5.6 Hz), 4.06 (m_{c}, 4H), 4.16 (q, 2H, ³*J*= 7.1 Hz), 4.55 (m_{c}, 1H), 6.74 (s, 1 H), 7.91 (d, 1H, ³*J*= 8.1 Hz), 9.98 (d, 1H, ³*J*= 7.8 Hz), 10.90 (s, 1H).
HRMS (ESI): *m*/*z* ber. für C₂₈H₅₀N₄O₈ [M + H]⁺: 571.37014 , gef.: 571.37034.
(3*R*,4*R*,5*S*)-4-Acetamido-5-(*N*-guanidino)-3-(1-ethylpropoxy)cyclohex-1-en-1-carbonsäure (**9**)

### A) Pharmakokinetische Charakterisierung der Hemmstoffe und Prodrugs

### 1. Stabilitätsuntersuchungen für die Amidin-Wirkform (3) sowie deren Prodrugs (2, 4) und die Guanidin-Wirkform (9) sowie deren Prodrugs (6, 10) über 6h

Die Stabilitätsuntersuchungen wurden in 50 mM Kaliumphosphat Puffer in einer Konzentration von 0.2 mM durchgeführt. Dazu wurde eine 2 mM Stammlösung in 10 mM Kaliumphosphat Puffer pH 7.4 hergestellt und 1:10 mit Phosphatpuffer des jeweiligen pH-Wertes verdünnt. Jede Verbindung wurde bei pH 2.0, 7.4 und 9.0 untersucht. Dazu wurde alle 30 min eine Probe per HPLC analysiert und die Stabilität über 6h untersucht. Die Konzentration bei t=0 min wurde gleich 100% gesetzt.

Zusätzlich wurden die Substanzen in humanem und murinem Plasma untersucht. Dazu wurden 630 µL des Plasmas mit 70 µL einer 2 mM Stammlösung der jeweiligen Verbindung in 10 mM Phosphatpuffer pH 7.4 versetzt. Die Inkubationen wurden in einem Schüttelwasserbad bei 37°C durchgeführt. Beendet wurde die Inkubation zu den Zeitpunkten, 15, 30, 45, 60, 90 und 120 min durch Entnahme von 100 µL Probe und Zugabe von 100 µL Acetonitril. Die Proben wurden zentrifugiert (12.000 rpm / 10 min) und der Überstand per HPLC vermessen.

Die Stabilitätsuntersuchungen wurden mit Hilfe folgender HPLC-Methode ausgewertet.

**HPLC-Methode**

| | | | |
|---|---|---|---|
| HPLC-System | Waters Autosampler 717plus, Waters 600 Controller, Waters 600 Pump, Waters 2487 Dual λ Absorbance Detector und EZChrom Elite Client/Server Aufnahme- und Auswertesoftware (Version 2.8.3) | | |
| Säule: | LiChrospher 60 RP-select B (125x4mm, 5 µm) mit einer RP-select B Vorsäule (4x4 mm). | | |
| Fluss: | 1 ml/min | | |
| Fliessmittel: | für **3, 9** | 60% | 10 mM KH₂PO₄ / 0.1% TFA pH 3.0 |
| | | 40% | MeOH |
| | für **2, 4, 6, 10** | 50% | 10 mM KH₂PO₄ / 0.1 % TFA pH 3.0 |
| | | 50% | MeOH |
| Laufzeit: | 7.5 min | | |
| Detektion: | 230 nm | | |
| Injektionsvolumen: | 10 µl | | |
| Retentionszeiten: | **4** | 4.2 ± 0.1 min | |
| | **9** | 4.4 ± 0.2 min | |
| | **3** | 4.5 ± 0.2 min | |
| | **6** | 4.6 ± 0.1 min | |
| | **2** | 4.8 ± 0.1 min | |
| | **10** | 3.7 ± 0.1 min | |

### Stabilitätsuntersuchungen für die Amidin-Wirkform (3) und Guanidin-Wirkform (9) über 2 Wochen

### Untersuchungen bei pH 7.4:

Die Lagerstabilität in gelöster Form wurde in einer Konzentration von 0,2 mg/ml bestimmt. Dazu wurde die Verbindung in 50 mM KH₂PO₄-Puffer pH 7,4 bzw. Aqua Bidest angelöst und über den Untersuchungszeitraum bei RT (pH 7.4) oder im Kühlschrank bei 4°C (pH 7.4 bzw. Aqua Bidest) gelagert. Es wurde die Konzentration der Wirkform nach 12h, 1d, 2d, 4d, 7d und 14d mit Hilfe der HPLC bestimmt.

### 2. Löslichkeitsuntersuchungen der untersuchten Verbindungen

### Ermittlung der Löslichkeit bei unterschiedlichen pH Werten:

Die Löslichkeit der Verbindungen wurde in Phosphatpuffer bei unterschiedlichen pH-Werten (2.0, 7.4 und 9.0) bestimmt. Dazu wurden einige mg der Verbindungen eingewogen und mit dem Volumen 50 mM KH₂PO₄-Puffer des jeweiligen pH-Wertes für eine 50 mM Lösung versetzt. Sofern sich die Verbindung nicht vollständig gelöst hatte, wurde die Suspension für 30 min geschüttelt. Anschließend wurde der ungelöste Anteil bei 10.000 RPM für 15 min abzentrifugiert und die Konzentration im Überstand mit Hilfe der HPLC bestimmt.

### 3. Bestimmung der Proteinbindung der Amidin-Wirkform (3) sowie deren Prodrugs (2, 4) und der Guanidin-Wirkform (9) sowie deren Prodrugs (6, 10)

Die Plasmaprotein-Bindung wurde in drei verschiedenen Konzentrationen (10, 25 und 50 µM) durchgeführt. Als Proteinlösungen wurde eine 4%ige Albumin-Lösung verwendet. Es wurden jeweils 50 µl einer 10fach konzentrierten Substanz-Lösung in 450 µl der Proteinlösung pipettiert. Die Inkubation erfolgte über 15 min im Schüttelwasserbad bei 37°C. Im Anschluss wurden die Proben in Ultrafiltrationseinheiten (Vivaspin 500, 10 kDa cut off) überführt und für 15 min bei 10.000 RPM zentrifugiert. Das Filtrat wurde per HPLC analysiert. Zusätzlich wurde für jede Konzentration eine Kontrolle durchgeführt, die nicht mit Protein versetzt und nicht zentrifugiert wurde. Eine weitere Kontrolle ohne Proteinzusatz, die jedoch durch die Filtrationseinheit abzentrifugiert wurde, zeigte, dass das die Prodrugs nicht von der Membran zurückgehalten werden und dienten der Validierung der Methodik.

Zusätzlich wurde die Proteinbindung der beiden Wirkformen Oseltamivir-Amidin (**3**) und Oseltamivir-Guanidin (**9**) in humanem Plasma untersucht. Dazu wurde anstelle der 4%igen Albumin-Lösung humanes Plasma verwendet. Es wurden Proteinbindung von 3.7 ± 1.4% für Verbindung **3** und 8.6 ± 3.0% für Verbindung **9** ermittelt. Die Werte liegen erwartungsgemäß etwas über den mit der 4%igen-Albumin-Lösung erhaltenen Werten und sind auf das Vorhandensein weiterer Plasmaproteine (z.B. α₁-saures Glykoprotein) neben dem Albumin zurückzuführen.

### 4. Untersuchung der Bioaktivierung der verschiedenen Prodrugs (2, 4, 6, 10)

### Ermittlung der Aktivierung der Prodrugs mit verschiedenen subzellulären Enzymsystemen:

Die Aktivierung der Prodrugs in ihre Wirkformen wurde *in vitro* mittels subzellulärer Enzympräparationen bestimmt. Als Enzympräparationen wurden 9000g Überstände, Mikrosomen und Mitochondrien aus humanen und porcinen Lebergeweben verwendet. Die Inkubationsansätze setzten sich aus 500 µM Prodrug, 500 µM NADH, 1 U Esterase und 0.3 mg Enzympräparation, gelöst in 150 µl 100 mM Phosphatpuffer pH 6.3, zusammen. Die Inkubation erfolgte über 30 min bei 37°C im Schüttelwasserbad. Beendet wurde die Inkubation durch Zugabe von 150 µl Acetonitril. Anschließend wurden die Proben 10 min geschüttelt und das ausgefällte Protein bei 10.000 RPM für 15 min abzentrifugiert. Der Überstand wurde mit Hilfe der HPLC vermessen.

**HPLC-Methode zur Bestimmung der Wirkformen (3/9) neben den Prodrugs (2, 4 / 6, 10)**

| | | |
|---|---|---|
| HPLC-System | Waters Alliance HPLC-System mit Waters e2695 XC Separations Modul, Waters 2998 Photodiode Array Detector und Empower 2 Software | |
| Säule: | LiChrospher 60 RP-select B (125x4mm, 5 µm) mit C18 Vorsäule (4x4mm) | |
| Fluss: | 1 ml/min | |
| Fliessmittel: | 70% | 10 mM KH₂PO₄ / 0.1% TFA pH 6.5 |
| | 30% | MeOH |
| Laufzeit: | 12 min | |
| Detektion: | 210 nm | |
| Injektionsvolumen: | 10 µl | |
| Retentionszeit: | **3** | 4.8 ± 0.2 min |
| | **9** | 4.7 ± 0.2 min |
| | **10** | 24.8 ± 0.4 min |
| | **6** | 25.2 ± 0.3 min |
| | **4** | 26.6 ± 0.3 min |
| | **2** | 26.6 ± 0.3 min |

### Antivirale Wirksamkeit

### Bestimmung der antiviralen Wirkung im Chemilumineszenz-basierten Neuraminidase (NA)-Hemmtest

Folgende Influenza Viren wurden verwendet für die Studien:
**H1N1-Viren:** A/Jena/5258/2009, A/Jena/5555/09, A/HH/1580/09, A/342/2009(oseltamivir resistent)
**H2N3-Viren:** Hongkong/8/68, Sachsen/6/02, Berlin/10/04, Rheinl.Pfalz/3911/03.

Die Hemmung der viralen Neuraminidase durch die Prüfsubstanzen **3** und **9** sowie die Kontrollsubstanzen wurde mit dem kommerziell verfügbaren NA-Star Kit (Tropix, Applied Biosystems, Darmstadt) überprüft.

Entsprechend den Empfehlungen des Herstellers wurde in einem Vortest zunächst die optimale Verdünnung der Testviren für den nachfolgenden Hemmtest bestimmt. Dazu wurden die Virussuspensionen in NA-Star Buffer (Verdünnungsfaktor 3) in Abwesenheit der Neuraminidaseinhibitoren (NAI) verdünnt. Die Virusverdünnung, die zu einem Signal und Hintergrundverhältnis von 40 : 1 führte, wurde anschließend in den NA-Hemmtesten zur Bestimmung der 50%igen Hemmkonzentrationen eingesetzt.

Im NA-Hemmtest wurden für die 6 Viruskontrollen pro Platte 25 µl Testpuffer oder 25 µl der Prüf- (3 Parallelen pro Verdünnung) bzw. der Kontrollsubstanz (2 Parallelen pro Verdünnung) in Testpuffer in die einzelnen Vertiefungen der Mikrotestplatte mit 96 Vertiefungen appliziert. Danach erfolgte die Zugabe von 25 µl Virusverdünnung zu jeder Vertiefung. Nach einer 20-minütigen Inkubationszeit bei 37°C wurde das Substrat 1:500 in Testpuffer verdünnt und je 10 µl in jede Vertiefung gegeben. Die Chemilumineszenzmessung erfolgte 30 min später in einem Plattenmessgerät (Mikrotiterplate Luminometer, Dynex Technolgy). Für die Auswertung der Teste wurde der Mittelwert der gemessenen Chemilumineszenz der 6 unbehandelten Viruskontrollen als 100 % Wert für die NA-Aktivität angenommen und zur Berechnung der relativen NA-Aktivität der einzelnen Substanzbehandelten Vertiefungen eingesetzt. Anhand der erhaltenen mittleren Dosis-Wirkungskurve von 2 unabhängigen Testen wurde anschließend die 50%ige Hemmkonzentration (IC50) der Prüf- und Kontrollsubstanzen mittels linearer Interpolation im EXCEL berechnet.

### Bestimmung der antiviralen Wirkung der Testsubstanzen im Virusertrags (VE)-Hemmtest

| | |
|---|---|
| Zellen: | MDCK-Zellen |
| Influenzaviren: | a) A/Jena/5258/2009 (pandemisches H1N1; Oseltamivir-sensitiv) |
| | b) A/342/2009 (H1N1; Oseltamivir-resistent) |

Durch die Zugabe antiviral wirksamer Substanzen (100 µl/well; 3 Parallelen/ Konzentration/Prüfsubstanz und 2 Parallelen/Konzentration/Kontrollsubstanz, Verdünnungsfaktor 10) lässt sich die Virusreplikation gezielt hemmen. Dies kann anhand reduzierter Virustiter im Überstand experimentell festgestellt werden.

Im Test wurden 2-Tage-alte geschlossene Zellrasen mit einer Virusdosis beimpft, die 48 h nach Infektion zu einem inkompletten zytopathischen Effekt in den 3 unbehandelten Viruskontrollen führt. Nach einstündiger Inkubation bei 37°C wurde das nicht an die Zellen gebundene Virus durch 3-maliges Waschen der einzelnen Vertiefungen entfernt und 100 µl Testmedium (Zell- und Viruskontrollen) bzw. der Substanzverdünnungen zugegeben. Nach einer 48-stündigen Inkubation bei 37°C wurden die Überstände der einzelnen Vertiefungen für die nachfolgende Virustiterbestimmung abgenommen.

Die Bestimmung des Virustiters erfolgte in 2-Tage-alten MDCK-Zellrasen in Mikrotiterplatten. Von den Überständen aus dem VE-Hemmtest wurden dazu zunächst logarithmische Verdünnungsreihen (Verdünnungsfaktor 10; maximale Verdünnung 10⁻⁷) angelegt. Diese wurden auf Zellen (je 4 Vertiefungen/Virusverdünnung) überimpft und 4 Tage bei 37°C inkubiert. In dieser Zeit kam es zur Ausbildung des zytopathischen Effektes. Nach Fixierung und Färbung der Zellen mit einer Kristall-Violett-Formalin-Lösung erfolgte die visuelle Auswertung über einem Lichtkasten.

Im Anschluss daran wurden die Virustiter nach Reed und Muench berechnet. Der Mittelwert der Virustiter der 3 Viruskontrollen wurde bei der Berechnung der Titerreduktion als 100% angenommen.

### Bestimmung der antiviralen Wirkung der Testsubstanzen im zpE-Hemmtest

Die Replikation der im Test verwendeten Viren führt durch einen stark ausgeprägten zytopathischen Effekt (zpE) zur kompletten Zerstörung der Wirtszellen. Durch die Zugabe antiviral wirksamer Substanzen (100 µl/well; 3 Parallelen/Konzentration, Verdünnungsfaktor 2) lässt sich der Virusinduzierte zpE gezielt hemmen. Im Test wurden unbehandelte und Substanz-behandelte geschlossene Zellrasen mit einer Virusdosis beimpft, die 48 h nach Infektion zu einem vollständigen zpE in den unbehandelten Viruskontrollen führt. Zu diesem Zeitpunkt wurden die noch adhärenten Zellen mit einer Kristallviolett/Formalin Lösung fixiert und gefärbt. Die Hemmung des Virus-induzierten zpE wurde nach Farbstoffelution photometrisch in einem Dynatech-Plattenreader quantifiziert.

Die Berechnung der antiviralen Wirkung erfolgte durch einen Vergleich der optischen Dichten Substanz-behandelter und unbehandelter, virusinfizierter Zellen mit der durchschnittlichen optischen Dichte der Zellkontrollen, die als 100 % gesetzt wurde. Anhand der mittleren Dosis-Wirkungskurve von 2 Experimenten wurde mittels linearer Interpolation im EXCEL die Verdünnung berechnet, die die Ausbildung des Virus-induzierten zpE zu 50 % verhinderte (IC50).

### Zytotoxizitätstest zur Bestimmung der 50 % zytotoxischen Dosis (CC₅₀) der Prüfsubstanzen in MDCK (Madin darby canine kidney)-Zellrasen

MDCK-Zellen wurden in Mikrotiterplatten ausgesät und im Brutschrank bei 5 % CO₂, 37 °C und 95 % Luftfeuchte zur Ausbildung eines geschlossenen Zellrasens 48 h inkubiert. Danach wurde das Medium entfernt und die Substanzen in verschiedenen Konzentrationen (100 µl/well-, 3 Parallelen/Konzentration, Verdünnungsfaktor 2) in Kulturmedium aufgetragen. Für die Kontrollwertbestimmung (sechs unbehandelte Zellkontrollen) wurden jeweils 100 µl Medium eingesetzt. 72 h nach Substanzapplikation und Inkubation erfolgt die Färbung der Zellen mit Kristallviolett/Methanol. Nach dem Herauslösen des Farbstoffes wurde die optische Dichte (OD) der einzelnen Vertiefungen in einem Plattenphotometer der Firma Dynatech (550/630 nm) gemessen und mit dem Mittelwert der Zellkontrollen verglichen. Der Mittelwert der Kontrollen wurde als 100 % angenommen.

### Tierstudie

### Operation / Vorbereitung der Versuchstiere

Spraque Dawley (SD) Ratten wurden zur Habitation 10 Tage vor Versuchsbeginn mit einem Gewicht von ∼300-350 g geliefert und in einem klimatisierten Raum mit einer konstanten Temperatur von 20°C sowie einer Luftfeuchtigkeit von 50% gehalten. In diesem Raum herrschte ein Tag-Nacht-Rhythmus von jeweils zwölf Stunden. Die Dunkelphase begann täglich um 18 Uhr und ging um 6 Uhr in die Lichtphase über. Die Ratten wurden in Standardkäfigen der Größe 3 (Länge: 42 cm, Breite: 26 cm, Höhe: 15 cm) über die Eingewöhnungszeit gehalten und drei Tage vor Versuchsbeginn in einen extra Versuchsraum überführt, in dem identische Umweltbedingungen gegeben waren. Sie erhielten eine Haltungsdiät (Haltungsdiät für Ratten und Mäuse; Art.-Nr.: 1320; Altromin) sowie Leitungswasser ad libidum.

Die hier beschriebenen Tierexperimente wurden nach der "NIH-Guideline" und der entsprechenden Richtlinie zum Umgang und der Verwendung von Versuchstieren nach der Genehmigung durch das Ministerium für Landwirtschaft, Umwelt und ländliche Räume des Landes Schleswig-Holsteins durchgeführt.

Ratten, die eine i.v.-Applikation erhielten wurde in die Vene als auch in die Arteria femoralis ein Katheter implantiert. Ratten die nur eine orale Applikation von Substanzen erhielten nur einen Venen-Katheter.

Die Ratten wurden mit Pentobarbital (60 mg/kg i.p.) und bei eventuell unzureichender Narkosetiefe zusätzlich mit Diethylether narkotisiert. Nach Rasur des Nackenbereichs und der rechten Leistenregion wurden die Ratten zur Aufrechterhaltung der Körpertemperatur auf einen elektrischen Heiztisch (EBERLE, Typ 52102) in Rückenlage gebracht und die Hinterläufe fixiert. Längs der Leistenbeuge wurde ein ca. 1,5 cm langer Hautschnitt gesetzt. Anschließend wurde der Gefäßstrang aus Arteria femoralis, Vena femoralis und Nervus femoralis stumpf auf einer Länge von circa 1 cm frei präpariert.

Nach Separierung der Vena femoralis wurde um diese proximal ein Baumwollfaden gelegt und durch Straffung das Gefäß reversibel verschlossen. Ca. 5 mm in distaler Richtung liegend wurde mittels eines zweiten Fadens das Gefäß legiert, um so eine Blutstauung zu erzeugen. Mit einer Gefäßschere wurde in das Gefäß im Bereich der Stauung (ca. 1/3 der Gesamtstauungslänge von der distalen Ligatur entfernt) ein kleiner Schnitt gesetzt und ein mit Heparin-Lösung (250 I.E./ml) gefüllter Polyethylen-Schlauch (Länge: 26 cm; ID: 0,58 mm; AD: 0.96 mm) mit Hilfe eines Gefäßspreizers 3 cm bis in die Vena cava nach proximal eingeführt. Mit den proximal und distal liegenden Fäden wurde der Katheter am Gefäß fixiert.

Die Arteria femoralis wurde, im Gegensatz zur Vena femoralis, zuerst durch eine distale Ligatur verschlossen und danach proximal durch Straffung des Fadens gestaut. Auch hier wurde wie oben beschrieben ein Katheter implantiert. Aufgrund des geringen Innendurchmessers der Arterie wurde hierfür ein speziell angefertigter Arterienkatheter verwendet, der aus einem Polyethylen-Schlauch (Länge: 26 cm, ID: 0,58 mm, AD: 0.96 mm) sowie einem 3 cm langen angeschweißten Polyethylen-Schlauch (ID: 0,28 mm, AD: 0.61 mm) bestand.

Nach dem Einbinden der Katheter wurde das Tier in Bauchlage gebracht und ein ca. 5 mm breiter Hautschnitt im Nacken gesetzt. Mit Hilfe eines Metallstabes und eines Schlauches wurden die Katheter, die mit Drahtstiften verschlossen wurden, von der Leiste in den Nacken gezogen, im Nacken mit Baumwollfaden fixiert und auf ca. 3 cm Länge gekürzt.

Wieder in Rückenlage wurde zuerst das Unterhautfettgewebe und dann die Oberhaut mit drei bis vier doppelten Knopflochstichen zusammengenäht und mit Betaisadonna®-Lösung desinfiziert. An den Folgetagen wurden die Katheter morgens und abends mit je 300 µl Heparin-Lösung (250 I.E./ml) gespült. Die operierten Ratten wurden ab dem Tag der Katheterlegung einzeln in Versuchskäfigen aus Plexiglas mit den Maßen Höhe: 20 cm, Breite: 22 cm und Länge: 25 cm bzw. gehalten bzw. in Standardkäfigen der Größe 3.

Die katheterisierten Versuchstiere wurden nach der Operation für einen Tag in dem Versuchsraum und einzeln in ihren Versuchskäfigen gehalten. Die Applikation der Versuchsverbindungen erfolgte am zweiten Tage nach der Operation. Am Versuchstag wurden die Ratten 1 Stunde vor Versuchsbeginn gewogen und die Arterien-Katheter mit 300 µl Heparin-Lösung gespült. Im Anschluss erfolgte die i.v.- bzw. orale Applikation der Verbindungen.

### Durchführung der Tierstudie

Das Oseltamivir-Derivat 3 wurde 5 Ratten in einer Konzentration von 10 mg/kg intravenös verabreicht. Die orale Applikation der Neuraminidase Inhibitoren (2, 4) wurde an 5 bzw. 6 Ratten in einer Dosierung von 50 mg/kg durchgeführt. Zusätzlich wurde Derivat 3 (50 mg/kg) an 3 Ratten oral appliziert. Die oralen Applikationen wurden als Suspension bzw. Lösung mit Arabischem Gummi (10% m/V) per Schlundsonde vorgenommen.

Das Oseltamivir-Derivat **9** wurde 5 Ratten in einer Konzentration von 10 mg/kg intravenös verabreicht. Die orale Applikation der Neuraminidase Inhibitoren (**6, 10**) wurde an 4 bzw. 5 Ratten in einer Dosierung von 50 mg/kg durchgeführt. Zusätzlich wurde Derivat **9** (50 mg/kg) an 3 Ratten oral appliziert. Die oralen Applikationen wurden als Suspension bzw. Lösung mit Arabischem Gummi (10% m/V) per Schlundsonde vorgenommen.

Nach iv-Gabe wurden Plasmaproben nach 5, 10, 20, 45, 90, 150, 240 und 360 min abgenommen bzw. nach oraler Applikation nach 30, 60, 90, 120, 180, 240 und 360 min. Hierzu wurden jeweils 300 µl Vollblut mit einer Insulinspritze entnommen und in EDTA beschichtete Microvetten CB 300 (Sarstedt, Nümbrecht) überführt. Nach jeder Abnahme wurde mit 100 µl 0,9%-iger Kochsalzlösung bzw. im Abstand von 60 min mit Heparin-Lösung (250 I.E./ml) gespült. Die Blutprobe wurde kurz geschüttelt und bis zur Zentrifugation (4°C; 14000 U/min; 10 min) auf Eis gestellt. Anschließend wurden die Proben bei -80°C eingefroren.

Die Tötung erfolgte durch Dekapitation 6 Stunden nach Medikamentengabe mit einer Guillotine. Im Anschluss wurden die Organe entnommen. Alle Organe wurden gesäubert und in mit Trockeneis gekühltem 2-Methylbutan gefroren. Es wurden Leber, Niere und Lunge entnommen.

### Analyse der Plasmaproben

Die Plasmaproben wurden aufgearbeitet und mit Hilfe der HPLC analysiert. Dazu wurden die Plasmaproben bei Raumtemperatur aufgetaut. Es wurden jeweils 80 µl Methanol (+ 0.2% TFA) vorgelegt und anschließend 80 µl der Plasmaproben zupipettiert. Die Proben wurden für 45 min geschüttelt, um Plasmaproteine zu fällen. Die Proben wurden bei -80°C eingefroren, aufgetaut und für weitere 15 min geschüttelt. Die Proben wurden für 15 min bei 13.000 RPM zentrifugiert und der Überstand in HPLC-Vials überführt. Jeweils 50 µl wurden für die Bestimmungen mittels LC/MS verwendet.

Die Tierstudien wurden mit Hilfe folgender LC/MS-Methode ausgewertet.

**LC/MS-Methode**

| | | | |
|---|---|---|---|
| HPLC-System: | Agilent 1100 Binary Pump, Agilent 1100 Diode Array Detector, Agilent 1100 Wellplate Autosampler, Degasser G1322A | | |
| Säule: | LiChrospher 60 RP-select B (125x3mm, 5 µm) mit einer RP-select B Vorsäule (4x4 mm) | | |
| Massenspektrometer: | Esquire-LC | | |
| Interface: | ESI (Elektronenstoß-Ionisation) | | |
| Nebulizer: | 40.0 psi | | |
| Dry Gas: | 8.0 ml/min | | |
| Dry Temperatur: | 350°C | | |
| HV-Capillary: | 5000 V | | |
| Fliessmittel: | A | 0.1% TFA in Aqua bidest (pH 2.5) | |
| | B | 0.1% TFA in MeOH | |
| Gradientenprofil: | Zeit | A [%] | B [%] |
| | 0 | 55 | 45 |
| | 8 | 25 | 75 |
| | 10 | 25 | 75 |
| | 11 | 55 | 45 |
| | 17 | 55 | 45 |
| Flussrate: | 0.3 ml/min | | |
| Laufzeit: | 17 min | | |
| Detektion: | PDA (190-400 nm) | | |
| Injektionsvolumen: | 50 µl | | |
| Retentionszeiten: | **3** | 5.1 ± 0.3 min | |
| | **9** | 5.1 ± 0.3 min | |
| | **11** | 5.2 ± 0.3 min | |
| | **12** | 5.2 ± 0.3 min | |

### Kurze Beschreibung der Abbildungen

- Abbildung 1:: Stabilität einiger erfindungsgemäßer Verbindungen bei unterschiedlichen pH-Werten in murinem bzw. humanem Plasma
- Abbildung 2:: Stabilität der Wirkformen A) **3** und B) **9** in verschiedenen Medien und bei verschiedenen Temperaturen.
- Abbildung 3:: Plasmaspiegel von Derivat 3 nach intravenöser Applikation von Derivat 3 in insgesamt 5 Ratten.
- Abbildung 4:: Plasmaspiegel von Derivat 3 nach oraler Applikation von Derivat 4
- Abbildung 5:: Plasmaspiegel von Derivat 3 und Metabolit 11 nach oraler Applikation von Derivat 4
- Abbildung 6:: Plasmaspiegel von Derivat 3 nach oraler Applikation von Derivat 3
- Abbildung 7:: Plasmaspiegel von Derivat 3 nach oraler Applikation von Derivat 2
- Abbildung 8:: Plasmaspiegel von Derivat 3 nach Applikation der Amidin-basierten Neuramidase Inhibitoren
- Abbildung 9:: Plasmaspiegel von Derivat 9 nach iv-Applikation von Derivat 9
- Abbildung 10:: Plasmaspiegel von Derivat 9 nach oraler Applikation von Derivat 9
- Abbildung 11:: Plasmaspiegel von Derivat 9 nach oraler Applikation von Derivat 6
- Abbildung 12:: Plasmaspiegel von Derivat 9 und Metabolit 12 nach oraler Applikation von Derivat 6
- Abbildung 13:: Plasmaspiegel von Derivat 9 nach Applikation der Guanidin-basierten Neuramidase Inhibitoren

### Literatur:

1. Hanessian, S.; Wang, J.; Montgomery, D.; Stoll, V.; Stewart, K. D.; Kati, W.; Maring, C.; Kempf, D.; Hutchins, C.; Laver, W. G. Design, synthesis, and neuraminidase inhibitory activity of GS-4071 analogues that utilize a novel hydrophobic paradigm. Bioorg Med Chem Lett 2002, 12, 3425-9.
2. Du, Q. S.; Wang, S. Q.; Chou, K. C. Analogue inhibitors by modifying oseltamivir based on the crystal neuraminidase structure for treating drug-resistant H5N1 virus. Biochem Biophys Res Commun 2007, 362, 525-31.
3. Masuda, T.; Shibuya, S.; Arai, M.; Yoshida, S.; Tomozawa, T.; Ohno, A.; Yamashita, M.; Honda, T. Synthesis and anti-influenza evaluation of orally active bicyclic ether derivatives related to zanamivir. Bioorg Med Chem Lett 2003, 13, 669-73.
4. Li, Y.; Zhou, B.; Wang, R. Rational design of Tamiflu derivatives targeting at the open conformation of neuraminidase subtype 1. J Mol Graph Model 2009, 28, 203-19.
5. D'Souza, C.; Kanyalkar, M.; Joshi, M.; Coutinho, E.; Srivastava, S. Search for novel neuraminidase inhibitors: Design, synthesis and interaction of oseltamivir derivatives with model membrane using docking, NMR and DSC methods. Biochim Biophys Acta 2009, 1788, 1740-51.
6. Wang, S. Q.; Cheng, X. C.; Dong, W. L.; Wang, R. L.; Chou, K. C. Three new powerful oseltamivir derivatives for inhibiting the neuraminidase of influenza virus. Biochem Biophys Res Commun 401, 188-91.
7. Carbain, B.; Collins, P. J.; Callum, L.; Martin, S. R.; Hay, A. J.; McCauley, J.; Streicher, H. Efficient synthesis of highly active phospha-isosteres of the influenza neuraminidase inhibitor oseltamivir. ChemMedChem 2009, 4, 335-7.
8. Shie, J. J.; Fang, J. M.; Wang, S. Y.; Tsai, K. C.; Cheng, Y. S.; Yang, A. S.; Hsiao, S. C.; Su, C. Y.; Wong, C. H. Synthesis of tamiflu and its phosphonate congeners possessing potent anti-influenza activity. J Am Chem Soc 2007, 129, 11892-3.
9. Smee, D. F.; Huffman, J. H.; Morrison, A. C.; Barnard, D. L.; Sidwell, R. W. Cyclopentane neuraminidase inhibitors with potent in vitro anti-influenza virus activities. Antimicrob Agents Chemother 2001, 45, 743-8.
10. Zhang, J.; Wang, Q.; Fang, H.; Xu, W.; Liu, A.; Du, G. Design, synthesis, inhibitory activity, and SAR studies of hydrophobic p-aminosalicylic acid derivatives as neuraminidase inhibitors. Bioorg Med Chem 2008, 16, 3839-47.
11. Albohy, A.; Mohan, S.; Zheng, R. B.; Pinto, B. M.; Cairo, C. W. Inhibitor selectivity of a new class of oseltamivir analogs against viral neuraminidase over human neuraminidase enzymes. Bioorg Med Chem 19, 2817-22.

## Patentansprüche

1. Verbindung gemäß der allgemeinen Strukturformel 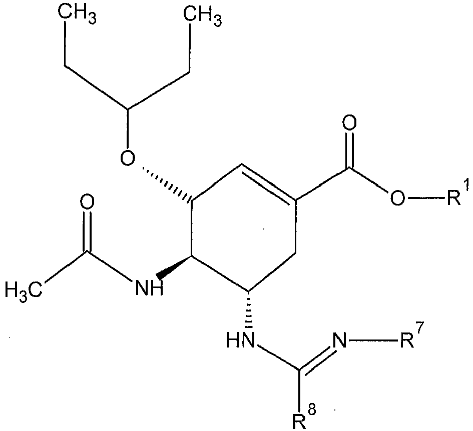 sowie pharmazeutisch verträgliche Salze, Solvate und/oder R/S Enatiomere hiervon, wobei
R¹ H, eine verzweigte oder unverzweigte, gesättigte oder ungesättigte, substituierte, oder nicht substituierte Kohlenwasserstoffkette mit einer Kettenlänge von 1 bis 12 ist,
R⁷ H oder OH ist,
R⁸ H, R⁹, NH₂, NHR⁹, N(R⁹)₂ oder NHCOOR¹ ist,
R⁹ ein verzweigter oder unverzweigter Alkylrest mit einer Kettenlänge von 1 bis 4 C-Atomen ist, und
wobei mögliche Substituenten für R¹ und R⁹, ausgewählt sind aus einer Gruppe bestehend aus Fluor, Chlor, Brom und Jod, Sauerstoff, Schwefel, Alkoxy-, Acyloxy-, Hydroxyl-, Mercapto-, Cyano-, Nitro- und Thioalkoxy-Gruppe,
wobei wenn R⁷ H und R⁸NH₂ ist, R¹ nicht H oder CH₂CH₃ ist.

2. Verbindung nach Anspruch 1, wobei die Verbindung Amidoxim (3*R*,4*R*,5*S*)-4-Acetamido-5-[*N*-(*N*'-hydroxy)acetimidamido]-3-(1-ethylpropoxy)cyclohex-1-en-1-carbonsäureethylester oder (3R,4R,5S)-4-Acetamido-5-(N-acetimidamido)-3-(1-ethylpropoxy)cyclohex-1-en-1-carbonsäure und/oder pharmazeutisch verträgliche Salze, Solvate und/oder R/S Enantiomere hiervon ist.

3. Verbindung nach Anspruch 1 oder 2 zur Verwendung als Arzneimittel.

4. Verbindung gemäß Anspruch 1 oder 2 zur Behandlung von Influenza Infektionen.

5. Verfahren zur Herstellung einer Verbindung nach Anspruch 1 oder 2 umfassend die Umsetzung von Oseltamivir gemäß der allgemeinen Strukturformel 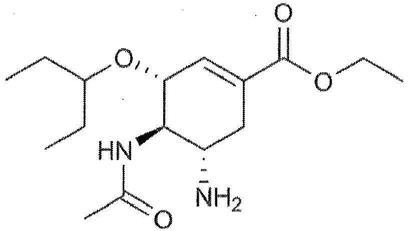 oder eines Derivates davon, welches mindestens den folgenden Schritt umfasst:
Umsetzung des Oseltamivirs mit einem Acylhydroximoylchlorid, in organischen Lösungsmitteln bei Raumtemperatur.

6. Verfahren nach Anspruch 5, wobei das organische Lösungsmittel ein Dichlormethan ist.

7. Verfahren zur Herstellung einer Verbindung nach einem der Ansprüche 1 bis 3 umfassend die Umsetzung von Oseltamivir (3*R*,4*R*,5*S*)-4-Acetamido-5-amino-3-(1-ethylpropoxy)cyclohex-1-en-1-carbonsäureethylester oder eines Derivates davon, welches mindestens einen der folgenden Schritte umfasst
i) Umsetzung des Oseltamivirs mit Bromcyan in einem organischen Lösungsmittel bei Raumtemperatur
ii) Umsetzung des in (i) gebildeten Cyanamids mit Hydroxylamin in Dioxan bei Raumtemperatur.

## Claims

1. A compound according to the general structural formula 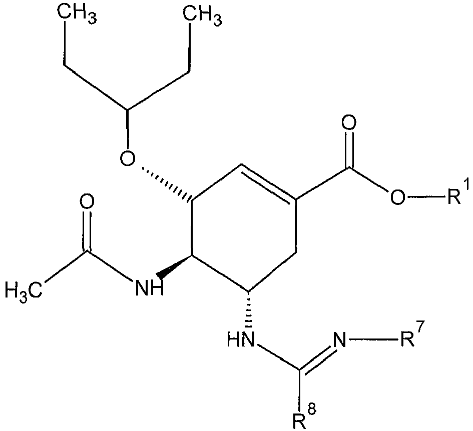 as well as pharmaceutically acceptable salts, solvates and/or R/S enantiomers thereof, wherein
R¹ is H, a branched or unbranched, saturated or unsaturated, substituted or unsubstituted hydrocarbon chain having a chain length of 1 to 12,
R⁷ is H or OH,
R⁸ is H or R⁹, NH₂, NHR⁹, N(R⁹)₂ or NHCOOR¹
R⁹ is a branched or unbranched alkyl group having a chain length of 1 to 4 carbon atoms, and
wherein possible substituents for R¹ and R⁹ are selected from the group consisting of fluorine, chlorine, bromine and iodine, oxygen, sulfur, alkoxy, acyloxy, hydroxyl, mercapto, cyano, nitro and a thio alkoxy group, wherein if R⁷ is H and R⁸ is NH₂, R¹ is not H or not CH₂CH₃.

2. Compound according to claim 1, wherein the compound is amidoxime (*3R,4R,5S*)-4-acetamido-5-[*N-(N'*-hydroxy)acetimidamido]-3-(1-ethylpropoxy)cyclohex-1-en-1-carboxylic acid ethyl ester
or (3R,4R,5S)-4-acetamido-5-(N-acetimidamido)-3-(1-ethylpropoxy)cyclohex-1-en-1-carboxylic acid and/or pharmaceutically acceptable salts, solvates and/or R/S enantiomers thereof.

3. Compound according to claim 1 or 2 for use as a pharmaceutical.

4. Compound according to claim 1 or 2 for the treatment of Influenza infections.

5. Method for preparing a compound according to claim 1 or 2, comprising the reaction of oseltamivir according to the general structural formula 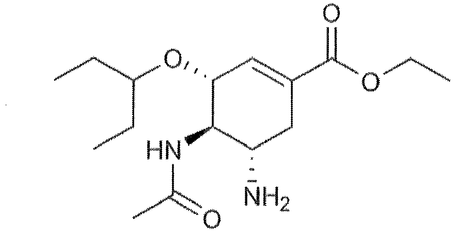 or a derivative thereof which comprises at least the following step:
reaction of oseltamivir with an acyl hydroximoyl chloride, in organic solvents at room temperature.

6. Method according to claim 5, wherein the organic solvent is a dichloromethane.

7. Method for preparing a compound according to 1 to 3, comprising the reaction of oseltamivir (*3R,4R,*5*S*)-4-acetamido-5-amino-3-(1-ethylpropoxy)cyclohex-1-en-1-carboxylic acid ethyl ester or a derivative thereof which comprises at least one of the following steps:
i) Reaction of oseltamivir with cyanogen bromide in an organic solvent at room temperature
ii) Reaction of the cyanamide formed in (i) with hydroxylamine in dioxane at room temperature.

## Revendications

1. Composé selon la formule structurelle générale 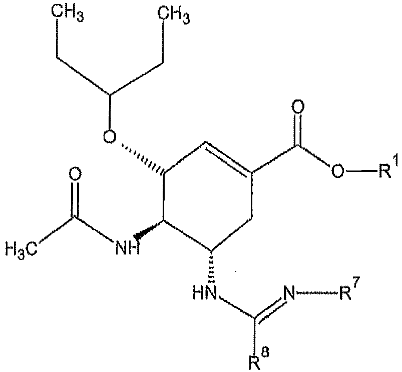 ainsi que sels pharmaceutiquement acceptables, solvants et/ou énantiomères R/S de ceux-ci,
dans lequel
R¹ est H, une chaîne hydrocarbonée ramifiée ou non ramifiée, saturée ou insaturée, substituée ou non substituée avec une longueur de chaîne de 1 à 12,
R⁷ est H ou OH,
R⁸ est H, R⁹, NH₂, NHR⁹, N(R⁹)₂ ou NHCOOR¹,
R⁹ est un radical alkyle ramifié ou non ramifié avec une longueur de chaîne de 1 à 4 atomes de C, et
dans lequel des substituants possibles pour R¹ et R⁹ sont choisis parmi un groupe constitué de fluor, de chlore, de brome et d'iode, d'oxygène, de soufre, de groupe alkoxy, acyloxy, hydroxyle, mercapto, cyano, nitro et thioalkoxy,
dans lequel quand R⁷ est H et R⁸ est NH₂, R¹ n'est pas H ou CH₂CH₃.

2. Composé selon la revendication 1, dans lequel le composé est de l'éthylester d'acide amidoxime (3R,4R,5S)-4-acétamido-5-[N-(N'-hydroxy)acétimidamido]-3-(1-éthylpropoxy)cyclohex-1-en-1-carboxylique ou de l'acide (3R,4R,5S)-4-acétamido-5-(N-acétimidamido)-3-(1-éthylpropoxy)cyclohex-1-en-1-carboxylique et/ou des sels pharmaceutiquement acceptables, des solvants et/ou des énantiomères R/S de ceux-ci.

3. Composé selon la revendication 1 ou 2 pour son utilisation en tant que médicament.

4. Composé selon la revendication 1 ou 2 pour le traitement d'infections grippales.

5. Procédé pour la préparation d'un composé selon la revendication 1 ou 2 comprenant la mise en oeuvre de l'oséltamivir selon la formule structurelle générale 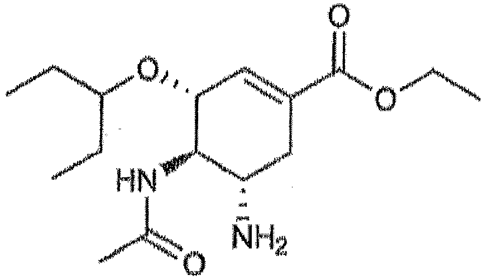 ou d'un dérivé de celui-ci, lequel comprend au moins l'étape suivante :
de mise en oeuvre de l'oséltamivir avec un chlorure acylhydroxymoyle dans des solvants organiques à température ambiante.

6. Procédé selon la revendication 5, dans lequel le solvant organique est du dichlorométhane.

7. Procédé pour la préparation d'un composé selon l'une quelconque des revendications 1 à 3 comprenant la mise en oeuvre d'oséltamivir éthylester d'acide (3R,4R,5S)-4-acétamido-5-amino-3-(1-éthylpropoxy)cyclohex-1-en-1-carboxylique ou d'un dérivé de celui-ci, lequel comprend au moins une des étapes suivantes
i) la mise en oeuvre de l'oséltamivir avec du bromure de cyanogène dans un solvant organique à température ambiante
ii) la mise en oeuvre du cyanamide formé lors de (i) avec de l'hydroxylamine dans du dioxane à température ambiante.
